# EUROPEAN PATENT SPECIFICATION

(11) **EP 3 288 924 B1**
(45) Date of publication and mention of the grant of the patent: **03.04.2019**
(21) Application number: 16720095.5
(22) Date of filing: 27.04.2016
(51) Int. Cl.: C07D 241/42, A01N 43/42, A01N 43/60, A01N 43/90, C07D 495/04

(54) **HERBICIDAL COMPOUNDS**
HERBIZIDE VERBINDUNGEN
COMPOSÉS HERBICIDES

(30) Priority: 30.04.2015 GB 201507467; 16.11.2015 GB 201520137
(43) Date of publication of application: 07.03.2018
(73) Proprietor: Syngenta Participations AG, 4058 Basel (CH)
(72) Inventor: SHANAHAN, Stephen Edward, Bracknell Berkshire RG42 6EY (GB); O'RIORDAN, Timothy Jeremiah Cornelius, Bracknell Berkshire RG42 6EY (GB)
(74) Representative: Syngenta International AG
(86) International application number: PCT/EP2016/059379
(87) International publication number: WO 2016/174073

(56) References cited:
- WO-A1-2010/130970
- WO-A1-2012/062531
- WO-A1-2015/173035

## Description

The present invention relates to herbicidal benzyloxy-substituted phenyl diones and benzyloxy-substituted phenyl-dioxo-thiazinone derivatives of formula (I), as well as to processes and intermediates used for the preparation of such derivatives. The invention further extends to herbicidal compositions comprising such derivatives, as well as to the use of such compounds and compositions in controlling undesirable plant growth: in particular the use in controlling weeds, such as broad-leaved dicotyledonous weeds, in crops of useful plants.

In addition, herbicidal diaza-naphthalene derivatives are known from WO2010/130970. Whilst WO2012/062531 discloses *5H*-quinoxaline-6-one derivatives, which exhibit herbicidal activity.

The present invention is based on the finding that benzyloxy-substituted phenyl diones and benzyloxy-substituted phenyl-dioxo-thiazinone derivatives of formula (I) exhibit surprisingly good herbicidal activity.

Thus, in a first aspect there is provided a compound of formula (I), 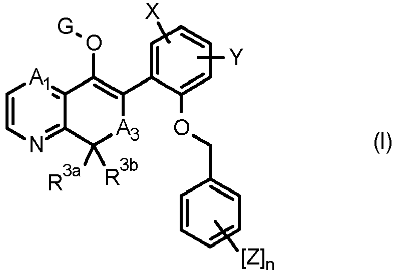
or a salt or N-oxide thereof;
wherein A₁ is CR¹ or N;
R¹ is hydrogen, C₁-C₄alkyl, C₁-C₄haloalkyl, C₁-C₄alkoxy, C₁-C₄alkylthio, halogen, cyano, or hydroxyl;
A₃ is C(O) or S(O)₂;
G is hydrogen, or C(O)R⁶;
X and Y are each independently hydrogen, C₁-C₃alkyl, C₁-C₃alkoxy, C₁-C₃haloalkyl, C₁-C₃haloalkoxy, or halogen;
n is an integer of 0, 1, 2, 3, 4, or 5;
each Z is independently C₁-C₃alkyl, C₁-C₃alkoxy, C₁-C₃haloalkyl, C₁-C₃haloalkoxy, or halogen;
R^{3a} and R^{3b} are independently hydrogen, halogen, cyano, C₁-C₈alkyl, C₁-C₈alkoxy-C₁-C₄alkyl-, C₁-C₈haloalkyl, C₂-C₈alkenyl, C₂-C₈haloalkenyl, C₂-C₈alkynyl, C₂-C₈haloalkynyl, C₃-C₁₀cycloalkyl, C₃-C₁₀cycloalkyl-C₁-C₄alkyl-, heterocyclyl, heterocyclyl-C₁-C₄alkyl-, or C₁-C₈alkoxycarbonyl-; or R^{3a} and R^{3b} together with the carbon atom they are attached to join to form a 3- to 10-membered carbocyclic ring or a 4- to 10-membered heterocyclic ring;
R⁶ is selected from the group consisting of C₁-C₆alkyl, C₂-C₆alkenyl, C₂-C₆alkynyl, C₁-C₆alkyl-S-, -NR⁷R⁸ and phenyl optionally substituted by one or more R⁹;
R⁷ and R⁸ are independently selected from the group consisting of C₁-C₆alkyl and C₁-C₆alkoxy, or R⁷ and R⁸ together can form a morpholinyl ring; and,
R⁹ is selected from the group consisting of halogen, cyano, nitro, C₁-C₃alkyl, C₁-C₃haloalky, C₁-C₃alkoxy and C₁-C₃haloalkoxy; and
with the proviso that when A₁ is CR¹, A₃ is C(O).

Compounds of formula (I) may contain asymmetric centres and may be present as a single enantiomer, pairs of enantiomers in any proportion or, where more than one asymmetric centre are present, contain diastereoisomers in all possible ratios. Typically one of the enantiomers has enhanced biological activity compared to the other possibilities.

Similarly, where there are di-substituted alkenes, these may be present in (*E*)- or (*Z*)-form or as mixtures of both in any proportion.

Compounds of formula (I) may also contain axes of chirality, and may be present as single atropisomers, or pairs of atropisomers in any proportion.

Furthermore, compounds of formula (I) may be in equilibrium with alternative tautomeric forms. For example, a compound of formula (I-i), i.e. a compound of formula (I) wherein A₃ is C(O) and G is hydrogen, can be drawn in at least three tautomeric forms:

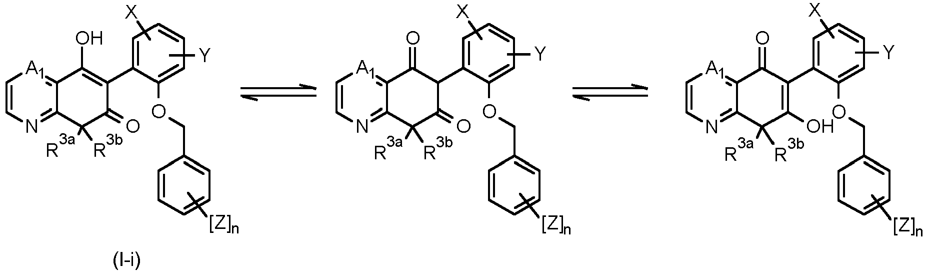

Similarly, a compound of formula (I-ii), i.e. a compound of formula (I) wherein A₃ is S(O)₂ and G is hydrogen, can be drawn in two tautomeric forms:

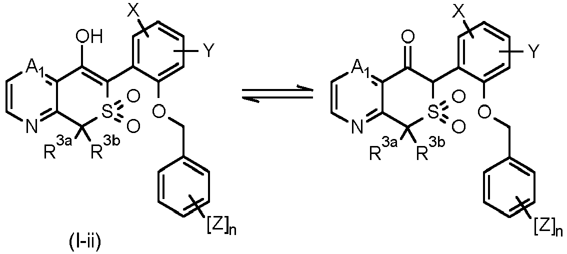

It should be appreciated that all tautomeric forms (single tautomer or mixtures thereof), racemic mixtures and single isomers are included within the scope of the present invention.

Each alkyl moiety either alone or as part of a larger group (such as alkoxy, alkylthio, alkoxycarbonyl, alkylcarbonyl, alkylaminocarbonyl, or dialkylaminocarbonyl, *et al.*) may be straight-chained or branched. Typically the alkyl groups are C₁-C₈alkyl groups (except where already defined more narrowly), but are preferably C₁-C₆alkyl, C₁-C₄alkyl or C₁-C₃alkyl groups, and, more preferably, are C₁-C₂alkyl groups (such as methyl). More specifically the alkyl group is, for example, methyl, ethyl, *n*-propyl, isopropyl, *n*-butyl, *sec*-butyl, isobutyl, *tert*-butyl, *n*-pentyl, neopentyl, or *n*-hexyl.

Alkenyl and alkynyl moieties can be in the form of straight or branched chains, and the alkenyl moieties, where appropriate, can be of either the (*E*)- or (*Z*)-configuration. Typically the alkenyl or alkynyl moieties are C₂-C₈alkenyl or C₂-C₈alkynyl, or C₂-C₆alkenyl or C₂-C₆alkynyl, but are preferably C₂-C₄alkenyl or C₂-C₄alkynyl, or C₂-C₃alkenyl or C₂-C₃alkynyl, more specifically vinyl, allyl, ethynyl, propargyl or prop-1-ynyl. Alkenyl and alkynyl moieties can contain one or more double and/or triple bonds in any combination; but preferably contain only one double bond (for alkenyl) or only one triple bond (for alkynyl).

The cycloalkyl groups generally refer to a C₃-C₁₀cycloalkyl moiety. Preferably, the term cycloalkyl refers to cyclopropyl, cyclobutyl, cyclopentyl or cyclohexyl.

In the context of the present specification the term "aryl" preferably means phenyl. The term "heteroaryl" as used herein means an aromatic ring system containing at least one ring heteroatom and consists of a single ring. Preferably, single rings will contain 1, 2 or 3 ring heteroatoms selected independently from nitrogen, oxygen and sulfur. Typically "heteroaryl" is furyl, thienyl, pyrrolyl, pyrazolyl, imidazolyl, 1,2,3-triazolyl, 1,2,4-triazolyl, oxazolyl, isoxazolyl, thiazolyl, isothiazolyl, 1,2,4-oxadiazolyl, 1,3,4-oxadiazolyl, 1,2,5-oxadiazolyl, 1,2,3-thiadiazolyl, 1,2,4-thiadiazolyl, 1,3,4-thiadiazolyl, 1,2,5-thiadiazolyl, pyridyl, pyrimidinyl, pyridazinyl, pyrazinyl, 1,2,3-triazinyl, 1,2,4-triazinyl, or 1,3,5-triazinyl.

Heterocyclyl groups and heterocyclic rings (either alone or as part of a larger group, such as heterocyclyl-alkyl-) are ring systems containing at least one heteroatom and can be in mono-cyclic form. Preferably, heterocyclyl groups will contain up to two heteroatoms which will preferably be chosen from nitrogen, oxygen and sulfur. Examples of heterocyclic groups include oxetanyl, thietanyl, azetidinyl and 7-oxa-bicyclo[2.2.1]hept-2-yl. Heterocyclyl groups containing a single oxygen atom as heteroatom are most preferred. The heterocyclyl groups are preferably 4- to 10-membred, more preferably 3- to 8-membered, and more preferably still 3- to 6-membered rings.

Halogen (or halo) encompasses fluorine, chlorine, bromine or iodine. The same correspondingly applies to halogen in the context of other definitions, such as haloalkyl or halophenyl.

Haloalkyl groups have a chain length from 1 to 8 carbon atoms, preferably from 1 to 6 carbon atoms, more preferably from 1 to 4 carbon atoms, and more preferably still from 1 to 3 carbon atoms. Such haloalkyl groups are, for example, fluoromethyl, difluoromethyl, trifluoromethyl, chloromethyl, dichloromethyl, trichloromethyl, 2,2,2-trifluoroethyl, 2-fluoroethyl, 2-chloroethyl, pentafluoroethyl, 1,1-difluoro-2,2,2-trichloroethyl, 2,2,3,3-tetrafluoroethyl and 2,2,2-trichloroethyl, heptafluoro-*n*-propyl and perfluoro-*n*-hexyl.

Alkoxy groups preferably have a chain length of from 1 to 8 carbon atoms, more preferably from 1 to 6 carbon atoms or 1 to 4 carbon atoms, and more preferably still from 1 to 3 carbon atoms. Alkoxy is, for example, methoxy, ethoxy, propoxy, isopropoxy, n-butoxy, isobutoxy, sec-butoxy or *tert*-butoxy or a pentyloxy or hexyloxy isomer, preferably methoxy and ethoxy. It should also be appreciated that two alkoxy substituents may be present on the same carbon atom.

Haloalkoxy is, for example, fluoromethoxy, difluoromethoxy, trifluoromethoxy, 2,2,2-trifluoroethoxy, 1,1,2,2-tetrafluoroethoxy, 2-fluoroethoxy, 2-chloroethoxy, 2,2-difluoroethoxy or 2,2,2-trichloroethoxy, preferably difluoromethoxy, 2-chloroethoxy or trifluoromethoxy.

C₁-C₆alkyl-S- (alkylthio) is, for example, methylthio, ethylthio, propylthio, isopropylthio, *n-*butylthio, isobutyl-thio, *sec*-butylthio or *tert*-butylthio, preferably methylthio or ethylthio.

C₁-C₆alkyl-S(O)- (alkylsulfinyl) is, for example, methylsulfinyl, ethylsulfinyl, propylsulfinyl, isopropylsulfinyl, *n*-butylsulfinyl, isobutyl-sulfinyl, *sec*-butylsulfinyl or *tert*-butylsulfinyl, preferably methylsulfinyl or ethylsulfinyl.

C₁-C₆alkyl-S(O)₂- (alkylsulfonyl) is, for example, methylsulfonyl, ethylsulfonyl, propylsulfonyl, isopropylsulfonyl, *n*-butylsulfonyl, isobutylsulfonyl, *sec*-butylsulfonyl or *tert-*butylsulfonyl, preferably methyl-sulfonyl or ethylsulfonyl.

The present invention also includes agronomically acceptable salts that the compounds of formula (I) may form with amines (for example ammonia, dimethylamine and triethylamine), alkali metal and alkaline earth metal bases or quaternary ammonium bases. Among the alkali metal and alkaline earth metal hydroxides, oxides, alkoxides and hydrogen carbonates and carbonates used as salt formers, emphasis is to be given to the hydroxides, alkoxides, oxides and carbonates of lithium, sodium, potassium, magnesium and calcium, but especially those of sodium, magnesium and calcium. The corresponding trimethylsulfonium salt may also be used. The compounds of formula (I) according to the invention also include hydrates which may be formed during the salt formation.

Preferred values of A₁, A₃, R¹ R^{3a}, R^{3b}, R⁶, R⁷, R⁸, R⁹, G, X, Y, Z, and n are as set out below, and a compound of formula (I) according to the invention may comprise any combination of said values. The skilled man will appreciate that values for any specified set of embodiments may be combined with values for any other set of embodiments where such combinations are not mutually exclusive.

As defined above, A₁ is N or CR¹. In one set of embodiments, A₁ is N. In a further set of embodiments, A₁ is CR¹.

Where A₁ is CR¹, it is preferred that R¹ is selected from the group consisting of hydrogen, C₁-C₄alkyl, C₁-C₄haloalkyl, C₁-C₄alkoxy, C₁-C₄alkylthio, halogen, cyano, and hydroxyl.

More preferably, R¹ is hydrogen, C₁-C₃alkyl, C₁-C₃alkoxy, halogen, cyano, or hydroxyl. Even more preferably R¹ is hydrogen, C₁-C₃alkyl, C₁-C₃alkoxy, or halogen. More preferably still R¹ is hydrogen, fluoro, chloro, bromo, methyl, or methoxy.

In one set of embodiments R¹ is hydrogen, methyl, or methoxy. In a further set of embodiments R¹ is hydrogen, or methoxy.

R^{3a} and R^{3b} are independently hydrogen, halogen, cyano, C₁-C₈alkyl, C₁-C₈alkoxy-C₁-C₄alkyl-, C₁-C₈haloalkyl, C₂-C₈alkenyl, C₂-C₈haloalkenyl, C₂-C₈alkynyl, C₂-C₈haloalkynyl, C₃-C₁₀cycloalkyl, C₃-C₁₀cycloalkyl-C₁-C₄alkyl-, heterocyclyl, heterocyclyl-C₁-C₄alkyl-, or C₁-C₈alkoxycarbonyl-; or R^{3a} and R^{3b} together with the carbon atom they are attached to join to form a 3- to 10-membered carbocyclic ring or a 4- to 10-membered heterocyclic ring.

Preferably R^{3a} and R^{3b} are hydrogen, halogen, C₁-C₈alkyl, C₁-C₈haloalkyl or C₂-C₈alkynyl. Examples of preferred groups for R^{3a} and R^{3b} include fluoro, methyl, ethyl, difluoroethyl and propargyl, more preferably R^{3a} and R^{3b} are both methyl. In a further embodiment where R^{3a} and R^{3b} together with the carbon atom they are attached to join to form a carbocyclic ring, the carbocyclic ring is preferably cyclopropyl.

As stated herein, A₃ is either C(O) or S(O)₂. In one set of preferred embodiments A₃ is C(O). In another set of preferred embodiments A₃ is S(O)₂.

As described herein, G may be hydrogen or -C(O)-R⁶, and R⁶ is selected from the group consisting of C₁-C₆alkyl, C₂-C₆alkenyl, C₂-C₆alkynyl, C₁-C₆alkyl-S-, C₁-C₆alkoxy, -NR⁷R⁸ and phenyl optionally substituted by one or more R⁹. As defined herein, R⁷ and R⁸ are independently C₁-C₆alkyl or C₁-C₆alkoxy-; or they can together form a morpholinyl ring. Preferably R⁷ and R⁸ are each independently selected from the group consisting of methyl, ethyl, propyl, methoxy, ethoxy and propoxy. R⁹ is selected from the group consisting of halogen, cyano, nitro, C₁-C₃alkyl, C₁-C₃haloalkyl, C₁-C₃alkoxy and C₁-C₃haloalkoxy.

Preferably R⁶ is C₁-C₆alkyl, C₂-C₆alkenyl, C₂-C₆alkynyl, C₁-C₆alkoxy, C₁-C₆alkyl-S-, - NR⁷R⁸ or phenyl optionally substituted by one or more R⁹, wherein R⁷ and R⁸ together form a morpholinyl ring.

More preferably R⁶ is C₁-C₄alkyl, C₂-C₃alkenyl, C₂-C₃alkynyl, C₁-C₄alkoxy, or -NR⁷R⁸ wherein R⁷ and R⁸ together form a morpholinyl ring. Even more preferably R⁶ is C₁-C₄alkyl, C₂-C₃alkenyl, C₂-C₃alkynyl or C₁-C₃alkoxy. More preferably still R⁶ is isopropyl, *tert*-butyl, methyl, ethyl, propargyl or methoxy.

In one set of embodiments G is hydrogen or -C(O)-R⁶, wherein R⁶ is C₁-C₄alkyl, C₂-C₃alkenyl, C₂-C₃alkynyl or C₁-C₃alkoxy. In a further set of embodiments G is hydrogen or - C(O)-R⁶, wherein R⁶ is isopropyl, *tert*-butyl, methyl, ethyl, propargyl or methoxy. However, it is particularly preferred that G is hydrogen.

Preferably X is C₁-C₃alkyl, C₁-C₃alkoxy, C₁-C₃haloalkyl, C₁-C₃haloalkoxy, or halogen, more preferably C₁-C₃haloalkyl or halogen, more preferably still halogen, in particular fluoro, chloro or bromo. Most preferably X is fluoro or chloro.

In a particularly preferred set of embodiments X is *ortho* with respect to the bi-cyclic moiety, and is for example C₁-C₃haloalkyl or halogen.

Preferably Y is hydrogen, C₁-C₃alkyl, C₁-C₃alkoxy, C₁-C₃haloalkyl, C₁-C₃haloalkoxy, or halogen, more preferably C₁-C₃alkyl, C₁-C₃haloalkyl, or halogen, more preferably still halogen, in particular fluoro, chloro or bromo. Most preferably, Y is fluoro or chloro.

In a particularly preferred set of embodiments, Y is *ortho* with respect to the bi-cyclic moiety, and is for example hydrogen, C₁-C₃alkyl, C₁-C₃haloalkyl, or halogen.

As described herein, Z may be C₁-C₃alkyl, C₁-C₃alkoxy, C₁-C₃haloalkyl, C₁-C₃haloalkoxy, or halogen and n is an integer of 0, 1, 2, 3, 4, or 5. Accordingly, the benzyl moiety of formula (I) may be represented as follows wherein p denotes the point of attachment to the remainder of the molecule via the ether link:

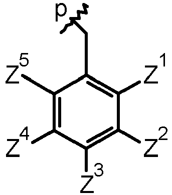

Preferably each Z radical is independently selected from halogen (in particular chloro), methyl, methoxy, and trifluoromethyl and trifluoromethoxy. More preferably each Z radical is independently selected from halogen (in particular chloro), methyl, methoxy, and trifluoromethoxy. Equally preferably, each Z radical is independently selected from halogen (in particular chloro), methyl, methoxy and trifluoromethyl.

It is preferred that n is 0, 1, or 2, more preferably 0 or 1. Where n is 1, it is preferred that Z is *para* with respect to the methoxy linker (i.e. Z is at position Z³). Where n is 2, it is preferred that one substituent will be *para* and the other will be *meta* with respect to the methoxy linker (i.e. one Z radical will be at position Z² or Z⁴, and the other Z radical will be at position Z³).

In one particularly preferred set of embodiments n is 0 (i.e. positions Z¹, Z², Z³, Z⁴ and Z⁵ all carry hydrogen).

In a further particularly preferred set of embodiments n is 2, and each Z is independently halogen, preferably each Z is chloro.

In yet another particularly preferred set of embodiments A₁ is N or CR¹, wherein R¹ is C₁-C₃alkyl;
A₃ is C(O) or S(O)₂;
G is hydrogen;
X and Y are each independently halogen or C₁-C₃haloalkyl;
n is 0; and
R^{3a} and R^{3b} are each independently C₁-C₃alkyl, or together with the carbon atom to which they are joined form a 3- to 6-membered carbocyclic ring.

More preferably A₁ is N or CH;
A₃ is C(O) or S(O)₂;
G is hydrogen;
X is *ortho* with respect to the bicyclic moiety and is selected from fluorine, chlorine and trifluoromethyl;
Y is *ortho* with respect to the benzyloxy group and is chlorine; and
R^{3a} and R^{3b} are each methyl or together with the carbon atom to which they are joined form a cyclopropyl ring.

Most preferably A₁ is N or CH;
A₃ is C(O) or S(O)₂;
G is hydrogen;
X is *ortho* with respect to the bicyclic moiety and is selected from fluorine, chlorine and trifluoromethyl;
Y is *ortho* with respect to the benzyloxy group and is chlorine; and
R^{3a} and R^{3b} are each methyl.

The compounds of the present invention may be prepared according to the following schemes, in which the substituents A₁, A₃, R^{3a,} R^{3b}, R^{6,} R⁷, R⁸, R⁹, X, Y, Z, Z¹, Z², Z³, Z⁴, Z⁵, and n have (unless otherwise stated explicitly) the definitions described hereinbefore.

Certain compounds (I-i) of the present invention may be prepared from compounds of formula (2) as shown in Reaction scheme 1 or from compounds (I-iii), also of the present invention, as shown in Reaction scheme 16. Compounds (I-i) are compounds of formula (I) in which A₃ is C(O) and G is hydrogen. Compounds (I-iii) are compounds of formula (I) in which A₃ is C(O) and G is C(O)R⁶.

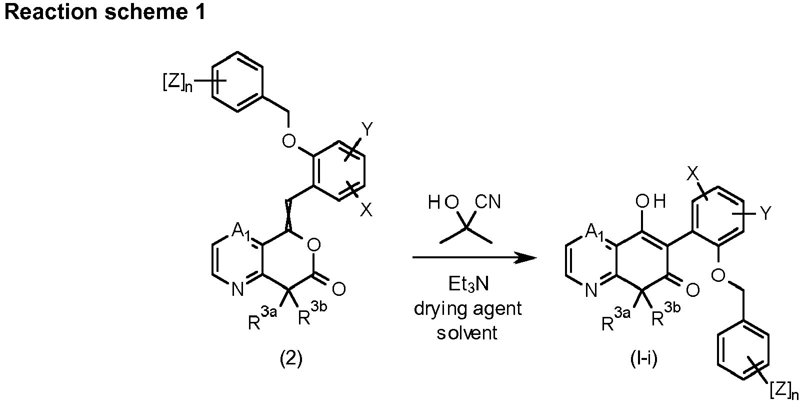

Compounds of formula (I-i) may be prepared by treatment of compounds (2) with acetone cyanohydrin in the presence of triethylamine, a suitable drying agent and a suitable solvent at a temperature between 0 °C and 60 °C. Examples of suitable drying agents include molecular sieves and magnesium sulfate. Examples of suitable solvents include acetonitrile and *N*,*N*-dimethylformamide.

Compounds (2) may be prepared from compounds (3) as shown in Reaction scheme 2.

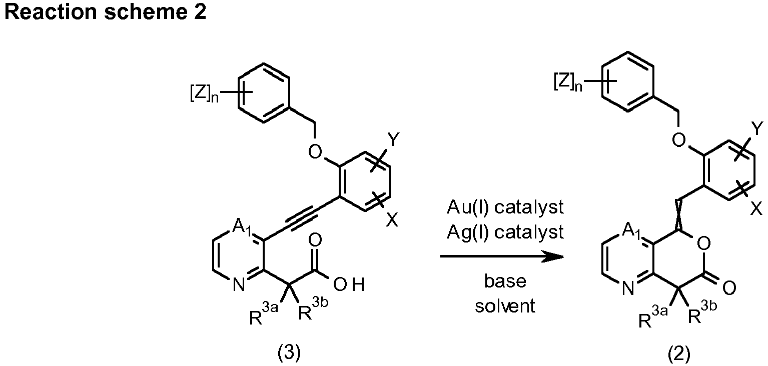

Compounds of formula (2) may be prepared by treatment of compounds (3) with a suitable Au(I) complex, optionally with the inclusion of a suitable Ag(I) complex and/or a suitable base, in the presence of a suitable solvent at a temperature between 20 °C and 100 °C. Examples of suitable Au(I) complexes include chloro(triphenylphosphine)gold(I) and gold(I) chloride. Examples of suitable Ag(I) complexes include silver tetrafluoroborate and silver nitrate. Examples of suitable bases include triethylamine and potassium carbonate. Examples of suitable solvents include carbon tetrachloride, acetonitrile and dichloromethane.

Compounds (3) may be prepared from compounds (4) as shown in Reaction scheme 3.

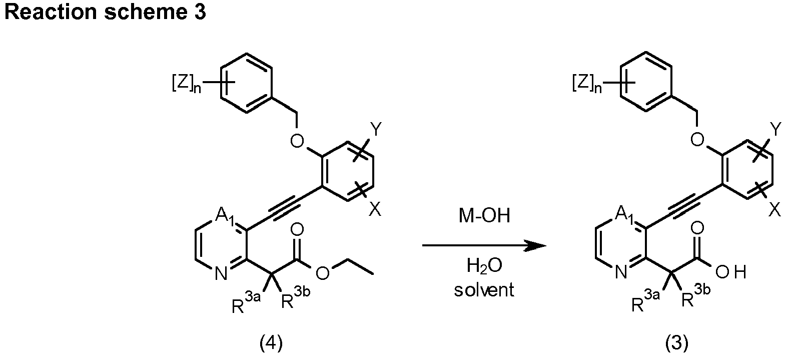

Compounds of formula (3) may be prepared by hydrolysis of compounds (4) with an alkali metal hydroxide in the presence of water and a suitable solvent a temperature between 20 °C and 100 °C. Examples of suitable alkali metal hydroxides are sodium hydroxide and potassium hydroxide. Examples of suitable solvents are methanol and ethanol.

Compounds (4) may be prepared from compounds (6) and compounds (5), where Q is chloro, bromo or iodo, as shown in Reaction scheme 4.

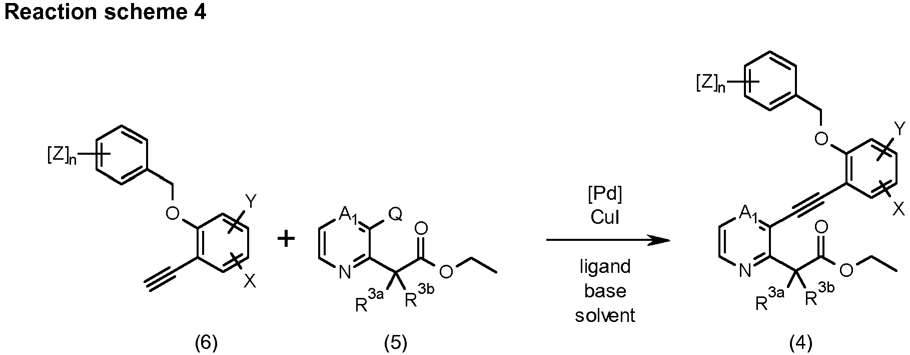

Compounds of formula (4) may be prepared by reaction of compounds (5) and compounds (6) in the presence of a suitable palladium complex, a suitable base and a suitable solvent, optionally with the inclusion of copper(I) iodide and/or a suitable ligand, at a temperature between 20 °C and 180 °C. Microwave heating or conventional heating may be used.

Examples of suitable palladium complexes include [1,1'-bis(diphenylphosphino)ferrocene]dichloropalladium(II), tetrakis(triphenylphosphine)palladium(0) and bis(triphenylphosphine)palladium(II) dichloride. Examples of suitable ligands include 4,5-bis(diphenylphosphino)-9,9-dimethylxanthene (Xantphos) and 2-dicyclohexylphosphino-2',4',6-triisopropylbiphenyl (Xphos). Examples of suitable bases include caesium carbonate and piperidine. Examples of suitable solvents include acetonitrile and dimethylsulfoxide.

Compounds (5a), a subset of compounds (5) where R³ = R^{3a} = R^{3b} and Q is chloro, bromo or iodo, may be prepared from compounds (8) [where Q is chloro, bromo or iodo] and electrophiles (7), where LG is a suitable leaving group such as iodide, chloride or trifluoromethane sulfonate, as shown in Reaction scheme 5. Compounds (5), where R^{3a} ≠ R^{3b}, may be prepared as shown in Reaction scheme 6.

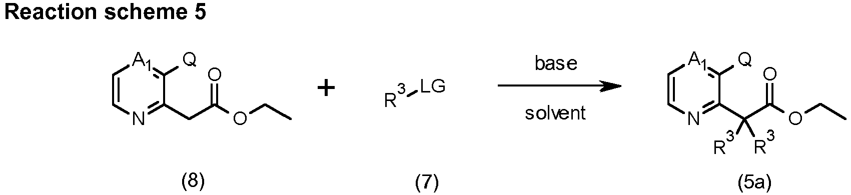

Compounds of formula (5a) may be prepared by reaction of compounds (8) with 2 or more molar equivalents of electrophiles (7) in the presence of a suitable base and suitable solvent at a temperature between -78 °C and 25 °C. Examples of suitable bases include sodium hydride, lithium bis(trimethylsilyl)amide, sodium bis(trimethylsilyl)amide and potassium bis(trimethylsilyl)amide. Examples of suitable solvents include *N*,*N*-dimethylformamide and tetrahydrofuran. With reference to Reaction scheme 5, many electrophiles (7) are commercially available. Examples are iodomethane, 1,2-dibromoethane and iodoethane.

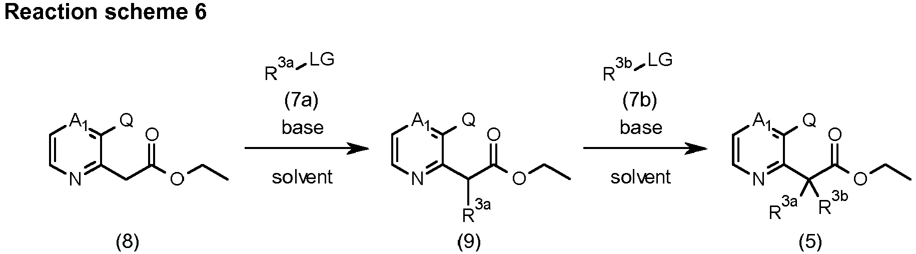

With reference to Reaction scheme 6, Q is chloro, bromo or iodo, and LG is a suitable leaving group such as iodide, chloride or trifluoromethane sulfonate. Compounds of formula (5) may be prepared by reaction of compounds (8) with electrophiles (7a) in the presence of a suitable base and suitable solvent at a temperature between -78 °C and 25 °C to provide compounds (9), which may be reacted with electrophiles (7b) under the same conditions to provide compounds (5), optionally isolating compounds (9) or by subsequent addition of reagents to provide compounds (5) directly. Compounds (9) may be isolated and used to prepare compounds of the invention (I) where R^{3b} is hydrogen. Examples of suitable bases include sodium hydride, lithium bis(trimethylsilyl)amide, sodium bis(trimethylsilyl)amide and potassium bis(trimethylsilyl)amide. Examples of suitable solvents include *N,N-*dimethylformamide and tetrahydrofuran. With reference to Reaction scheme 6, many electrophiles (7a) and (7b) are commercially available. Examples are iodomethane and iodoethane.

Compounds (8) may be prepared from compounds (10) as shown in Reaction scheme 7.

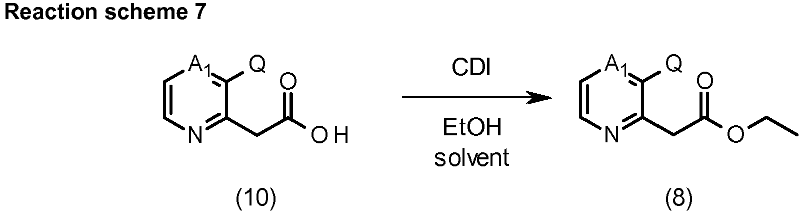

With reference to Reaction scheme 7, Q is chloro, bromo or iodo. Compounds of formula (8) may be prepared by treatment of compounds (10) with 1,1'-carbonyldiimidazole (CDI) and ethanol in the presence of a suitable solvent at a temperature between 0 °C and 40 °C. Examples of suitable solvents include *N*,*N*-dimethylformamide and dichloromethane.

Compounds (10) may be prepared from compounds (11) as shown in Reaction scheme 8.

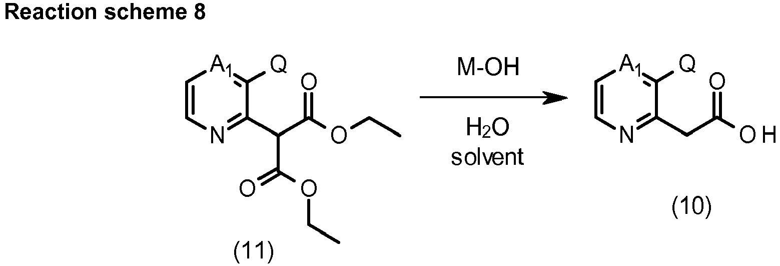

With reference to Reaction scheme 8, Q is chloro, bromo or iodo. Compounds of formula (10) may be prepared by treatment of compounds (11) with an alkali metal hydroxide in the presence of water and a suitable solvent at a temperature between 20 °C and 100 °C. Examples of suitable alkali metal hydroxides are sodium hydroxide and potassium hydroxide. Examples of suitable solvents are methanol and ethanol.

Compounds (11) may be prepared from compounds (13), where J is bromo, chloro or fluoro, as shown in Reaction scheme 9.

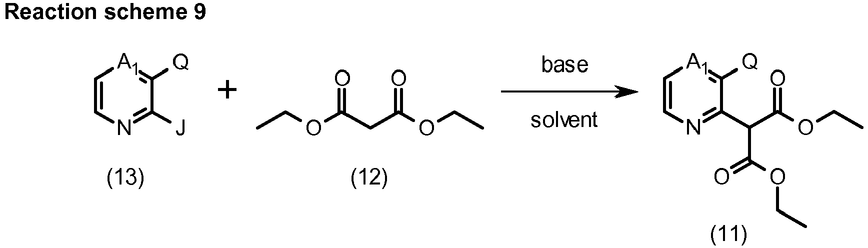

With reference to Reaction scheme 9, Q is chloro, bromo or iodo. Compounds of formula (11) may be prepared by treatment of compounds (13) with diethylmalonate (12) in the presence of a suitable base and suitable solvent at a temperature between 50 °C and 150 °C. Examples of suitable bases include potassium carbonate, sodium hydride and caesium carbonate. Examples of suitable solvents include *N*,*N*-dimethylformamide and toluene. With reference to Reaction scheme 9, many compounds (13) are commercially available. Examples are 2,3-dichloropyrazine, 2-bromo-3-chloropyridine and 3-chloro-2-fluoropyridine.

Compounds (6) may be prepared from compounds (14) as shown in Reaction scheme 10.

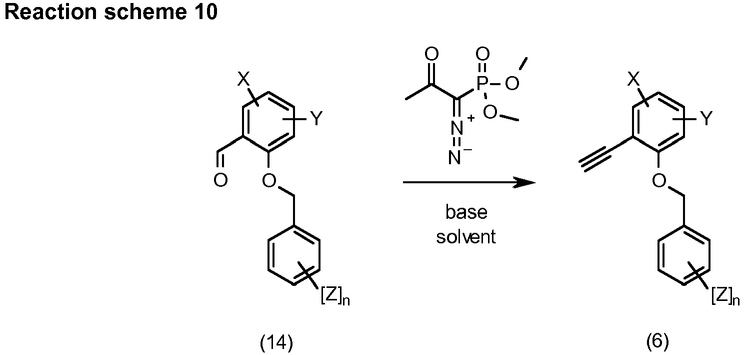

Compounds of formula (6) may be prepared by treatment of compounds (14) with dimethyl (1-diazo-2-oxopropyl)phosphonate in the presence of a suitable base and suitable solvent at a temperature between -20 °C and 25 °C. Examples of suitable bases include potassium carbonate and caesium carbonate. Examples of suitable solvents include methanol, tetrahydrofuran, acetonitrile and mixtures thereof. In a variation on Reaction scheme 10, the dimethyl (1-diazo-2-oxopropyl)phosphonate may be generated *in-situ* by reaction of tosyl azide with 1-dimethoxyphosphorylpropan-2-one [also known as dimethyl acetylmethylphosphonate, CAS number: 4202-14-6], mediated by the aforementioned solvent and base.

Compounds (14) may be prepared from compounds (16) as shown in Reaction scheme 11.

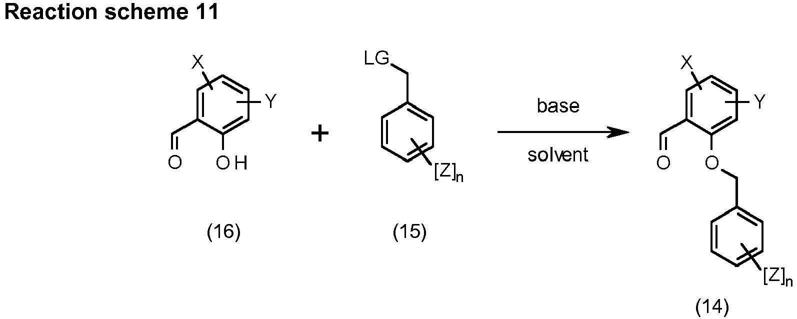

Compounds of formula (14) may be prepared by treatment of compounds (16) with benzyl electrophiles (15) in the presence of a suitable base and suitable solvent at a temperature between 20 and 70 °C. Examples of suitable bases include potassium carbonate and sodium hydroxide. Examples of suitable solvents include acetone, *N*,*N*-dimethylformamide, tetrahydrofuran and mixtures thereof. With reference to Reaction scheme 11 many benzyl electrophiles (15) are commercially available such as benzyl bromide, 3-chlorobenzyl bromide and 2-chloro-4-fluorobenzyl bromide.

Compounds (16) may be prepared from compounds (17) as shown in Reaction scheme 12.

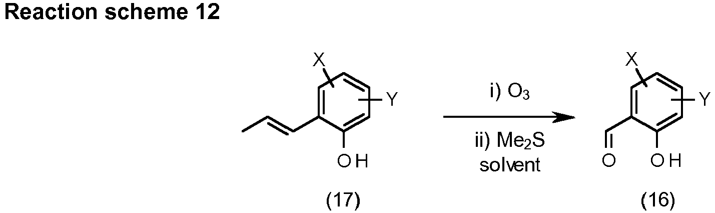

Compounds of formula (16) may be prepared by treatment of compounds (17) with ozone then dimethyl sulfide in the presence of a suitable solvent at a temperature between -78 °C and 20 °C. Examples of suitable solvents include methanol, dichloromethane and mixtures thereof.

Compounds (17) may be prepared from compounds (18) as shown in Reaction scheme 13.

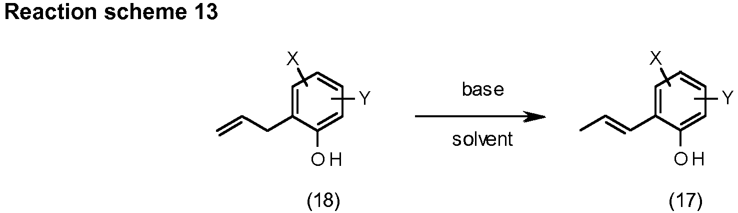

Compounds (17) may be prepared by treatment of compounds (18) with a suitable base in the presence of a suitable solvent at a temperature between 20 °C and 80 °C. Examples of suitable bases include lithium *tert*-butoxide and potassium *tert*-butoxide. Examples of suitable solvents include *N*,*N*-dimethylformamide and dimethylsulfoxide.

Compounds (18) may be prepared from compounds (19) as shown in Reaction scheme 14.

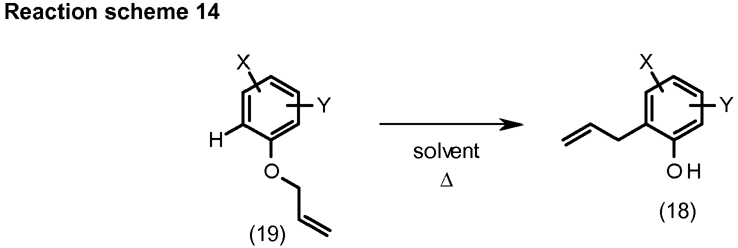

Compounds (18) may be prepared by heating compounds (19) in the presence of a solvent [such as *N*,*N*-dimethylformamide or 1-methylpyrrolidin-2-one], at a temperature between 180 °C and 220 °C. With reference to Reaction scheme 14, an example of compounds (19) is 2-allyloxy-1,4-dichloro-benzene, prepared according to J. Chem. Soc., Perkin Trans. 2, 2001, 1824. Other compounds (19) may be prepared similarly, according to Reaction scheme 15.

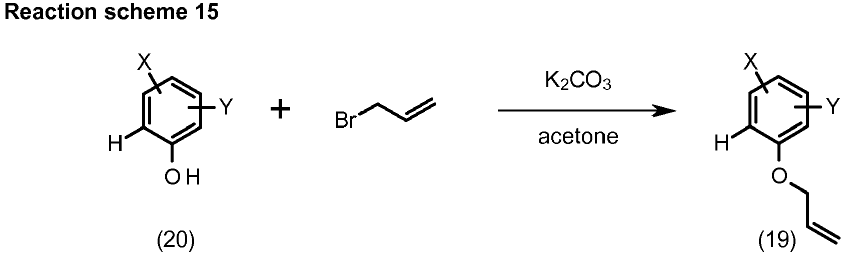

Compounds (19) may be prepared by treatment of compounds (20) with allyl bromide in the presence of potassium carbonate and acetone, at a temperature between 20 °C and 70 °C. With reference to Reaction scheme 15, many phenol compounds (20) are commercially available. Examples are 2,5-dichlorophenol and 2-chloro-5-fluorophenol.

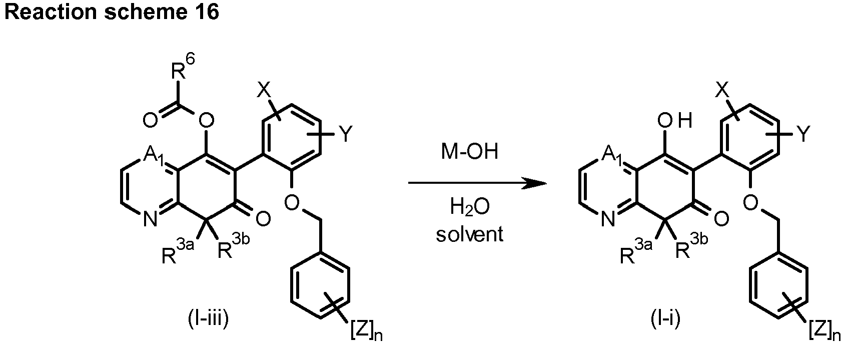

Compounds of formula (I-i) may be prepared by hydrolysis of compounds (I-iii) with an alkali metal hydroxide in the presence of water and a suitable solvent at a temperature between 20 °C and 100 °C. Examples of suitable alkali metal hydroxides are sodium hydroxide and potassium hydroxide. Examples of suitable solvents are methanol and ethanol.

Compounds (I-iii) may be prepared from compounds (I-i) as shown in Reaction scheme 17.

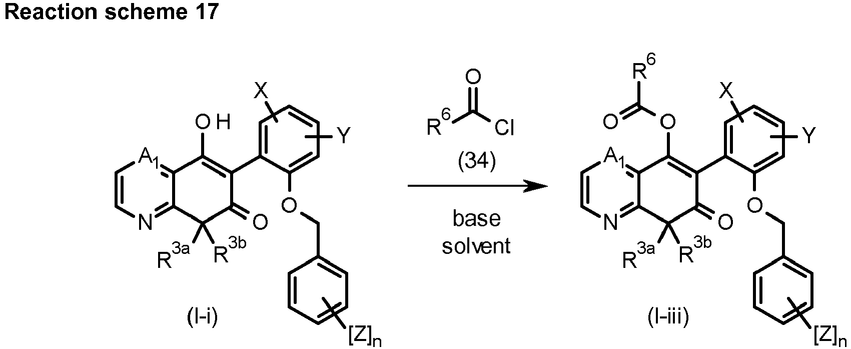

Compounds of formula (I-iii) may be prepared by treatment of compounds (I-i) with acyl chlorides (34) in the presence of a suitable base and a suitable solvent. Examples of a suitable base are pyridine and triethylamine. Examples of suitable solvents are dichloromethane and acetonitrile. With reference to Reaction scheme 17, many acyl chlorides (34) are commercially available, such as acetyl chloride and isobutyryl chloride.

Certain compounds (I-ii) of the present invention may be prepared from compounds of formula (21) as shown in Reaction scheme 18. Compounds (I-ii) are compounds of formula (I) in which A₃ is S(O)₂ and G is hydrogen.

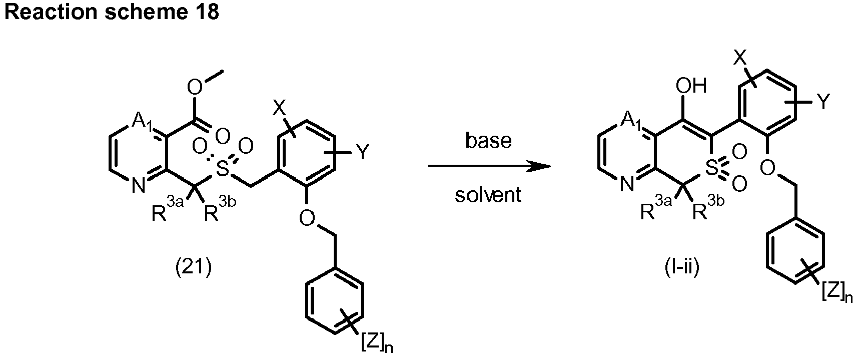

Compounds (I-ii) may be prepared by treatment of compounds (21) with a suitable base in the presence of a suitable solvent at a temperature between 0 °C and 150 °C. Examples of suitable bases include potassium carbonate, potassium *tert-*butoxide, potassium *tert*-pentoxide and lithium hexamethyldisilazide. Examples of suitable solvents include toluene, *N*,*N-*dimethylformamide and tetrahydrofuran.

Compounds (21a), a subset of compounds (21) where R³ = R^{3a} = R^{3b}, may be prepared from compounds (22) and electrophiles (7), where LG is a suitable leaving group such as iodide, chloride or trifluoromethane sulfonate, and R³ = R^{3a} = R^{3b}, as shown in Reaction scheme 19. Compounds (21), where R^{3a} ≠ R^{3b}, may be prepared from compounds (22) and electrophiles (7a) and/or (7b) as shown in Reaction scheme 20.

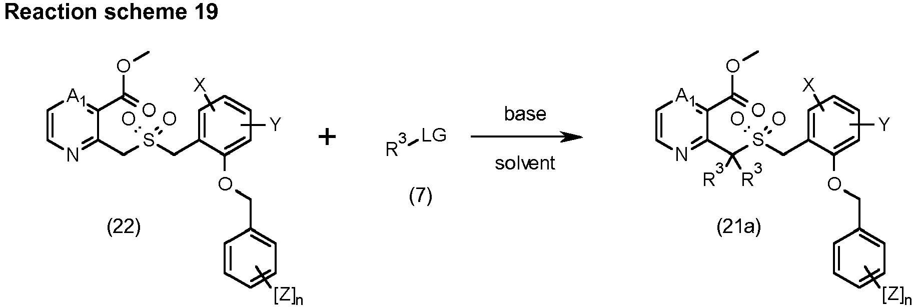

Compounds of formula (21a) may be prepared by reaction of compounds (22) with electrophiles (7) in the presence of a suitable base and suitable solvent at a temperature between -78 °C and 25 °C. Examples of suitable bases include sodium hydride, lithium bis(trimethylsilyl)amide, sodium bis(trimethylsilyl)amide, potassium *tert-*butoxide, potassium *tert-*pentoxide and potassium bis(trimethylsilyl)amide. Examples of suitable solvents include *N*,*N-*dimethylformamide and tetrahydrofuran. With reference to Reaction scheme 19, many electrophiles (7) are commercially available. Examples are iodomethane, 1,2-dibromoethane and iodoethane.

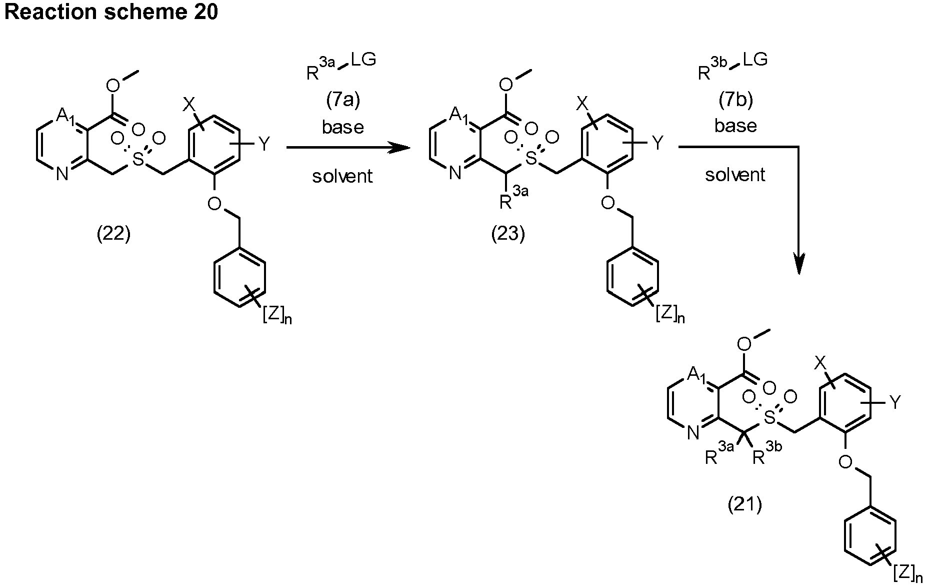

With respect to Reaction scheme 20, LG is a suitable leaving group such as iodide, chloride or trifluoromethane sulfonate. Compounds of formula (21) may be prepared by reaction of compounds (22) with electrophiles (7a) in the presence of a suitable base and suitable solvent at a temperature between -78 °C and 25 °C to provide compounds (23), which may be reacted with electrophiles (7b) under the same conditions to provide compounds (21), optionally isolating compounds (23) or by subsequent addition of reagents to provide compounds (21) directly. Compounds (23) may be isolated and used to prepare compounds of the invention (I) where R^{3b} is hydrogen. Examples of suitable bases include sodium hydride, lithium bis(trimethylsilyl)amide, sodium bis(trimethylsilyl)amide, potassium *tert-*butoxide, potassium *tert-*pentoxide and potassium bis(trimethylsilyl)amide. Examples of suitable solvents include *N*,*N-*dimethylformamide and tetrahydrofuran. With reference to Reaction scheme 20, many electrophiles (7a) and (7b) are commercially available. Examples are iodomethane and iodoethane.

Since the reactions described in Reaction schemes 18, 19 and 20 can on occasions be facilitated by the same pairings of solvent and base, it is sometimes observed that the methods described in Reaction schemes 19 and 20 give compounds (I-ii) directly [a 'one pot' or 'telescoped' process].

Compounds (22) may be prepared from compounds (24) as shown in Reaction scheme 21.

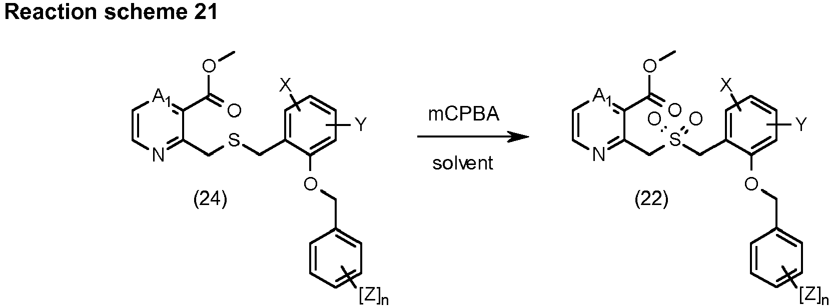

Compounds of formula (22) may be prepared by treatment of compounds (24) with m-chloroperbenzoic acid (mCPBA) in a suitable solvent at a temperature between 0 °C and 40 °C. Examples of suitable solvents are dichloromethane and chloroform.

Compounds (24) may be prepared from compounds (26), where Pg is acetyl or methanimidamidyl and salts thereof, and compounds (25) as shown in Reaction scheme 22 or from compounds (33), where Pg is acetyl or methanimidamidyl and salts thereof, and compounds (28) as shown in Reaction scheme 29.

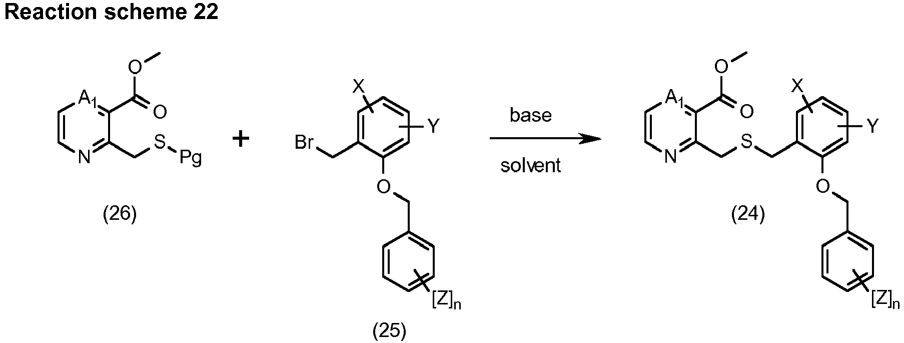

Compounds of formula (24) may be prepared by treatment of compounds (26) with compounds (25) in the presence of a suitable base and suitable solvent at a temperature between 20 °C and 150 °C. Microwave or conventional heating may be used. Examples of a suitable base are potassium carbonate, caesium carbonate and sodium hydroxide. Examples of suitable solvents include water, acetonitrile, methanol and ethanol.

Compounds (26) may be prepared from compounds (28) as shown in Reaction scheme 23.

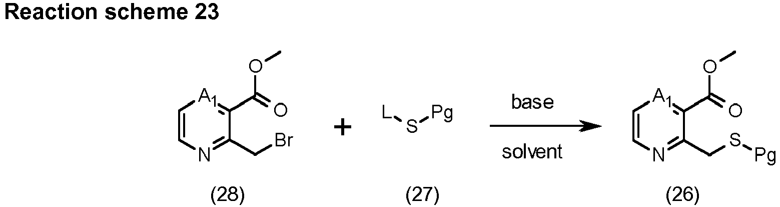

Compounds of formula (26) may be prepared by treatment of compounds (28) with sulfur nucleophiles (27) [where L is hydrogen or potassium, and Pg is acetyl or methanimidamidyl] in the presence of a suitable solvent and optionally in the presence of a base at a temperature between 20 °C and 150 °C. Microwave or conventional heating may be used. Examples of a suitable solvent are acetone, ethanol, tetrahydrofuran and dichloromethane. Examples of a suitable base are potassium carbonate and sodium hydroxide. Sulfur nucleophiles (27) can be obtained commercially [such as thiourea and potassium thioacetate].

Compounds (28) may be prepared from compounds (29) as shown in Reaction scheme 24.

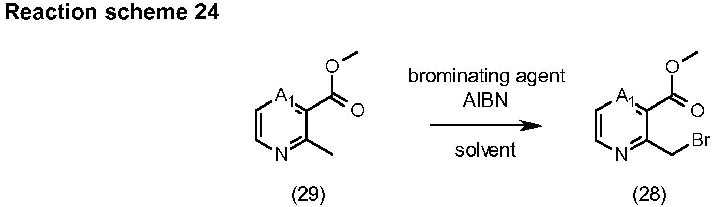

Compounds of formula (28) may be prepared by treatment of compounds (29) with a brominating agent and 2,2'-azobis(2-methylpropionitrile) (AIBN) in a suitable solvent at a temperature between 50 °C and 110 °C. Examples of suitable brominating agents are N-bromosuccinimide (NBS), bromine and 1,3-Dibromo-5,5-dimethylhydantoin (dibromantin). Examples of suitable solvents include carbon tetrachloride and benzotrifluoride.

Compounds (29) may be prepared from compounds (30) as shown in Reaction scheme 25.

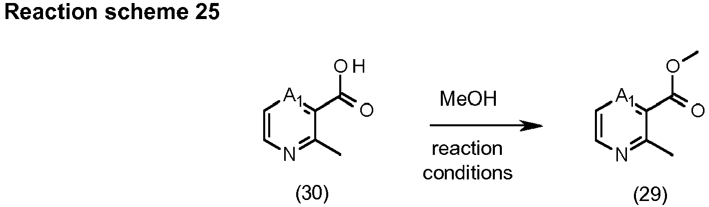

Compounds of formula (29) may be prepared by esterification of compounds (30) according to various known methods. For example, compounds (29) may be synthesized *via* prior activation of compounds (30) as their acyl chloride derivatives, and reaction with methanol. Alternatively, compounds (29) may be prepared by heating compounds (30) in methanol, at reflux temperature, in the presence of concentrated sulfuric acid. With reference to Reaction scheme 25 many carboxylic acids (30) are commercially available such as 3-methylpyrazine-2-carboxylic acid and 2-methylpyridine-3-carboxylic acid.

Compounds (25) may be prepared from compounds (31) as shown in Reaction scheme 26.

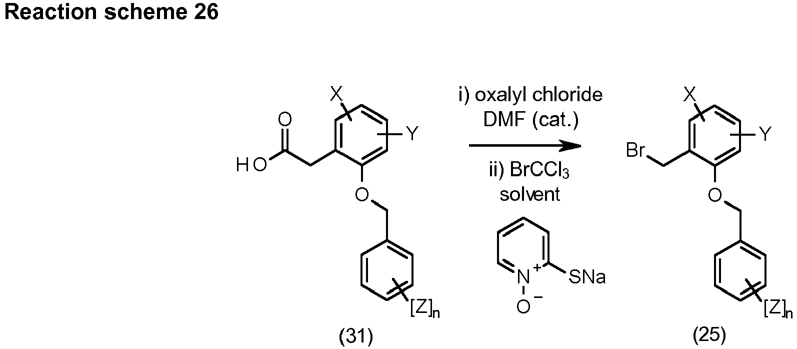

Compounds of formula (25) may be prepared by treatment of compounds (31) with oxalyl chloride, optionally with catalytic *N*,*N*-dimethylformamide, in the presence of a suitable solvent [such as dichloromethane, chloroform or benzene] to prepare the corresponding acyl chloride derivative, and subsequently treating the mixture with bromotrichloromethane and 2-mercaptopyridine *N*-oxide sodium salt, optionally with inclusion of a suitable solvent [such as dichloromethane, chloroform or benzene], optionally with heating, and optionally irradiating the reaction with UV light.

Compounds of formula (31) may be prepared from compounds (32) as shown in Reaction scheme 27.

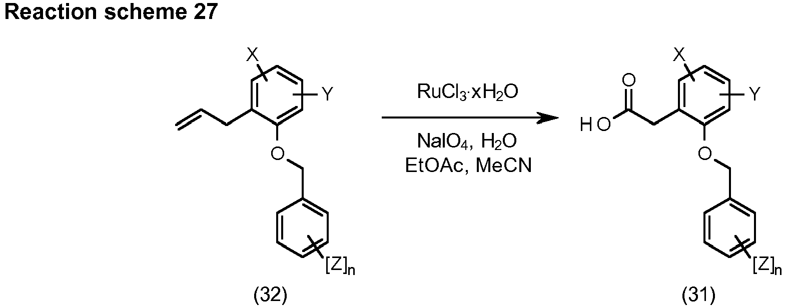

Compounds of formula (31) may be prepared by treatment of compounds (32) with ruthenium tetroxide, generated *in situ* from ruthenium trichloride hydrate and sodium metaperiodate, in a mixture of water, ethyl acetate and acetonitrile at a temperature between 0 °C and 40 °C.

Compounds of formula (32) may be prepared from compounds (18) as shown in Reaction scheme 28.

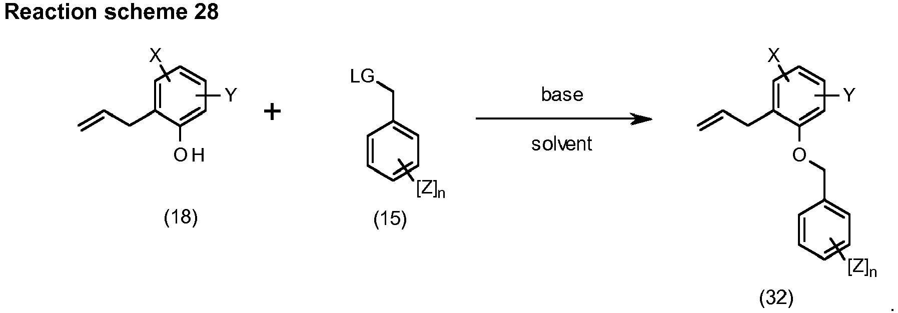

Compounds of formula (32) may be prepared by treatment of compounds (18) with benzyl electrophiles (15) [where LG is a suitable leaving group such as iodide, chloride or trifluoromethane sulfonate] in the presence of a suitable base and suitable solvent. Examples of suitable bases include potassium carbonate and sodium hydride. Examples of suitable solvents include acetone and *N*,*N*-dimethylformamide. With reference to Reaction scheme 28 many benzyl electrophiles (15) are commercially available such as benzyl bromide, 3-chlorobenzyl bromide and 2-chloro-4-fluorobenzyl bromide.

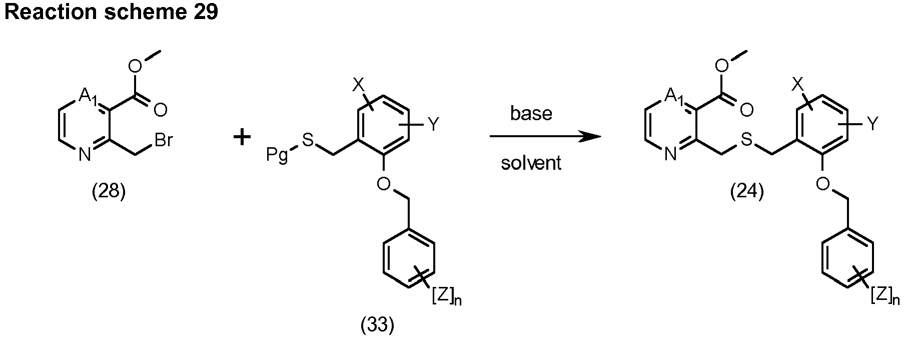

Compounds (24) may be prepared by treatment of compounds (28) with compounds (33) [where Pg is acetyl or methanimidamidyl] in the presence of a suitable base and suitable solvent at a temperature between 20 °C and 150 °C. Microwave or conventional heating may be used. Examples of a suitable base are potassium carbonate, caesium carbonate and sodium hydroxide. Examples of suitable solvents include water, acetonitrile, methanol and ethanol.

Compounds of formula (33) may be prepared from compounds (25) as shown in Reaction scheme 30.

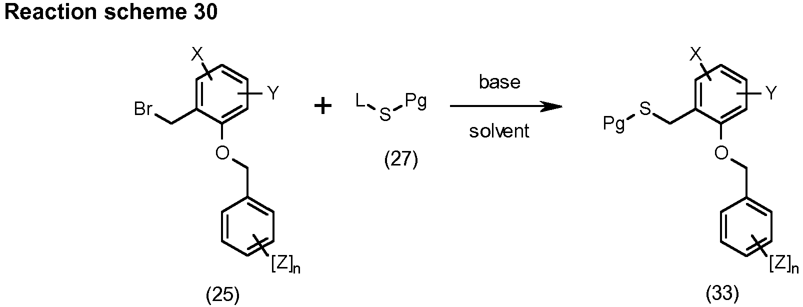

Compounds of formula (33) may be prepared by treatment of compounds (25) with sulfur nucleophiles (27) [where L is hydrogen or potassium, and Pg is acetyl or methanimidamidyl] in the presence of a suitable solvent and optionally in the presence of a base at a temperature between 20 °C and 150 °C. Microwave or conventional heating may be used. Examples of a suitable solvent are acetone, methanol, tetrahydrofuran and dichloromethane. Examples of a suitable base are potassium carbonate and sodium hydroxide. Many sulfur nucleophiles (27) are commercially available such as thiourea and potassium thioacetate.

The compounds according to the invention can be used as herbicidal agents in unmodified form, but they are generally formulated into compositions in various ways using formulation adjuvants, such as carriers, solvents and surface-active substances. The formulations can be in various physical forms, e.g. in the form of dusting powders, gels, wettable powders, water-dispersible granules, water-dispersible tablets, effervescent pellets, emulsifiable concentrates, micro-emulsifiable concentrates, oil-in-water emulsions, oil-flowables, aqueous dispersions, oily dispersions, suspo-emulsions, capsule suspensions, emulsifiable granules, soluble liquids, water-soluble concentrates (with water or a water-miscible organic solvent as carrier), impregnated polymer films or in other forms known e.g. from the Manual on Development and Use of FAO and WHO Specifications for Pesticides, United Nations, First Edition, Second Revision (2010). Such formulations can either be used directly or diluted prior to use. The dilutions can be made, for example, with water, liquid fertilisers, micronutrients, biological organisms, oil or solvents.

The formulations can be prepared e.g. by mixing the active ingredient with the formulation adjuvants in order to obtain compositions in the form of finely divided solids, granules, solutions, dispersions or emulsions. The active ingredients can also be formulated with other adjuvants, such as finely divided solids, mineral oils, oils of vegetable or animal origin, modified oils of vegetable or animal origin, organic solvents, water, surface-active substances or combinations thereof.

The active ingredients can also be contained in very fine microcapsules. Microcapsules contain the active ingredients in a porous carrier. This enables the active ingredients to be released into the environment in controlled amounts (e.g. slow-release). Microcapsules usually have a diameter of from 0.1 to 500 microns. They contain active ingredients in an amount of about from 25 to 95 % by weight of the capsule weight. The active ingredients can be in the form of a monolithic solid, in the form of fine particles in solid or liquid dispersion or in the form of a suitable solution. The encapsulating membranes can comprise, for example, natural or synthetic rubbers, cellulose, styrene/butadiene copolymers, polyacrylonitrile, polyacrylate, polyesters, polyamides, polyureas, polyurethane or chemically modified polymers and starch xanthates or other polymers that are known to the person skilled in the art. Alternatively, very fine microcapsules can be formed in which the active ingredient is contained in the form of finely divided particles in a solid matrix of base substance, but the microcapsules are not themselves encapsulated.

The formulation adjuvants that are suitable for the preparation of the compositions according to the invention are known *per se.* As liquid carriers there may be used: water, toluene, xylene, petroleum ether, vegetable oils, acetone, methyl ethyl ketone, cyclohexanone, acid anhydrides, acetonitrile, acetophenone, amyl acetate, 2-butanone, butylene carbonate, chlorobenzene, cyclohexane, cyclohexanol, alkyl esters of acetic acid, diacetone alcohol, 1,2-dichloropropane, diethanolamine, p-diethylbenzene, diethylene glycol, diethylene glycol abietate, diethylene glycol butyl ether, diethylene glycol ethyl ether, diethylene glycol methyl ether, *N*,*N*-dimethylformamide, dimethyl sulfoxide, 1,4-dioxane, dipropylene glycol, dipropylene glycol methyl ether, dipropylene glycol dibenzoate, diproxitol, alkylpyrrolidone, ethyl acetate, 2-ethylhexanol, ethylene carbonate, 1,1,1-trichloroethane, 2-heptanone, alpha-pinene, d-limonene, ethyl lactate, ethylene glycol, ethylene glycol butyl ether, ethylene glycol methyl ether, gamma-butyrolactone, glycerol, glycerol acetate, glycerol diacetate, glycerol triacetate, hexadecane, hexylene glycol, isoamyl acetate, isobornyl acetate, isooctane, isophorone, isopropylbenzene, isopropyl myristate, lactic acid, laurylamine, mesityl oxide, methoxypropanol, methyl isoamyl ketone, methyl isobutyl ketone, methyl laurate, methyl octanoate, methyl oleate, methylene chloride, *m*-xylene, *n*-hexane, *n*-octylamine, octadecanoic acid, octylamine acetate, oleic acid, oleylamine, *o*-xylene, phenol, polyethylene glycol, propionic acid, propyl lactate, propylene carbonate, propylene glycol, propylene glycol methyl ether, *p*-xylene, toluene, triethyl phosphate, triethylene glycol, xylenesulfonic acid, paraffin, mineral oil, trichloroethylene, perchloroethylene, ethyl acetate, amyl acetate, butyl acetate, propylene glycol methyl ether, diethylene glycol methyl ether, methanol, ethanol, isopropanol, and alcohols of higher molecular weight, such as amyl alcohol, tetrahydrofurfuryl alcohol, hexanol, octanol, ethylene glycol, propylene glycol, glycerol, *N*-methyl-2-pyrrolidone and the like.

Suitable solid carriers are, for example, talc, titanium dioxide, pyrophyllite clay, silica, attapulgite clay, kieselguhr, limestone, calcium carbonate, bentonite, calcium montmorillonite, cottonseed husks, wheat flour, soybean flour, pumice, wood flour, ground walnut shells, lignin and similar substances.

A large number of surface-active substances can advantageously be used in both solid and liquid formulations, especially in those formulations which can be diluted with a carrier prior to use. Surface-active substances may be anionic, cationic, non-ionic or polymeric and they can be used as emulsifiers, wetting agents or suspending agents or for other purposes. Typical surface-active substances include, for example, salts of alkyl sulfates, such as diethanolammonium lauryl sulfate; salts of alkylarylsulfonates, such as calcium dodecylbenzenesulfonate; alkylphenol/alkylene oxide addition products, such as nonylphenol ethoxylate; alcohol/alkylene oxide addition products, such as tridecylalcohol ethoxylate; soaps, such as sodium stearate; salts of alkylnaphthalenesulfonates, such as sodium dibutylnaphthalenesulfonate; dialkyl esters of sulfosuccinate salts, such as sodium di(2-ethylhexyl)sulfosuccinate; sorbitol esters, such as sorbitol oleate; quaternary amines, such as lauryltrimethylammonium chloride, polyethylene glycol esters of fatty acids, such as polyethylene glycol stearate; block copolymers of ethylene oxide and propylene oxide; and salts of mono- and di-alkylphosphate esters; and also further substances described e.g. in McCutcheon's Detergents and Emulsifiers Annual, MC Publishing Corp., Ridgewood New Jersey (1981).

Further adjuvants that can be used in pesticidal formulations include crystallisation inhibitors, viscosity modifiers, suspending agents, dyes, anti-oxidants, foaming agents, light absorbers, mixing auxiliaries, antifoams, complexing agents, neutralising or pH-modifying substances and buffers, corrosion inhibitors, fragrances, wetting agents, take-up enhancers, micronutrients, plasticisers, glidants, lubricants, dispersants, thickeners, antifreezes, microbicides, and liquid and solid fertilisers.

The compositions according to the invention can include an additive comprising an oil of vegetable or animal origin, a mineral oil, alkyl esters of such oils or mixtures of such oils and oil derivatives. The amount of oil additive in the composition according to the invention is generally from 0.01 to 10 %, based on the mixture to be applied. For example, the oil additive can be added to a spray tank in the desired concentration after a spray mixture has been prepared. Preferred oil additives comprise mineral oils or an oil of vegetable origin, for example rapeseed oil, olive oil or sunflower oil, emulsified vegetable oil, alkyl esters of oils of vegetable origin, for example the methyl derivatives, or an oil of animal origin, such as fish oil or beef tallow. Preferred oil additives comprise alkyl esters of C₈-C₂₂ fatty acids, especially the methyl derivatives of C₁₂-C₁₈ fatty acids, for example the methyl esters of lauric acid, palmitic acid and oleic acid (methyl laurate, methyl palmitate and methyl oleate, respectively). Many oil derivatives are known from the Compendium of Herbicide Adjuvants, 10th Edition, Southern Illinois University, 2010.

The herbicidal compositions generally comprise from 0.1 to 99 % by weight, especially from 0.1 to 95 % by weight, compounds of formula (I) and from 1 to 99.9 % by weight of a formulation adjuvant which preferably includes from 0 to 25 % by weight of a surface-active substance. The inventive compositions generally comprise from 0.1 to 99 % by weight, especially from 0.1 to 95 % by weight, of compounds of the present invention and from 1 to 99.9 % by weight of a formulation adjuvant which preferably includes from 0 to 25 % by weight of a surface-active substance. Whereas commercial products may preferably be formulated as concentrates, the end user will normally employ dilute formulations.

The rates of application vary within wide limits and depend on the nature of the soil, the method of application, the crop plant, the pest to be controlled, the prevailing climatic conditions, and other factors governed by the method of application, the time of application and the target crop. As a general guideline compounds may be applied at a rate of from 1 to 2000 l/ha, especially from 10 to 1000 l/ha.

Preferred formulations can have the following compositions (weight %):

### Emulsifiable concentrates:

| | |
|---|---|
| active ingredient: | 1 to 95 %, preferably 60 to 90 % |
| surface-active agent: | 1 to 30 %, preferably 5 to 20 % |
| liquid carrier: | 1 to 80 %, preferably 1 to 35 % |

### Dusts:

| | |
|---|---|
| active ingredient: | 0.1 to 10 %, preferably 0.1 to 5 % |
| solid carrier: | 99.9 to 90 %, preferably 99.9 to 99 % |

### Suspension concentrates:

| | |
|---|---|
| active ingredient: | 5 to 75 %, preferably 10 to 50 % |
| water: | 94 to 24 %, preferably 88 to 30 % |
| surface-active agent: | 1 to 40 %, preferably 2 to 30 % |

### Wettable powders:

| | |
|---|---|
| active ingredient: | 0.5 to 90 %, preferably 1 to 80 % |
| surface-active agent: | 0.5 to 20 %, preferably 1 to 15 % |
| solid carrier: | 5 to 95 %, preferably 15 to 90 % |

### Granules:

| | |
|---|---|
| active ingredient: | 0.1 to 30 %, preferably 0.1 to 15 % |
| solid carrier: | 99.5 to 70 %, preferably 97 to 85 % |

The following Examples further illustrate, but do not limit, the invention:

| **Wettable powders** | **a)** | **b)** | **c)** |
|---|---|---|---|
| active ingredients | 25 % | 50 % | 75 % |
| sodium lignosulfonate | 5 % | 5 % | - |
| sodium lauryl sulfate | 3 % | - | 5 % |
| sodium diisobutylnaphthalenesulfonate | - | 6 % | 10 % |
| phenol polyethylene glycol ether (7-8 mol of ethylene oxide) | - | 2 % | - |
| highly dispersed silicic acid | 5 % | 10 % | 10 % |
| Kaolin | 62 % | 27 % | - |

The combination is thoroughly mixed with the adjuvants and the mixture is thoroughly ground in a suitable mill, affording wettable powders that can be diluted with water to give suspensions of the desired concentration.

| **Powders for dry seed treatment** | **a)** | **b)** | **c)** |
|---|---|---|---|
| active ingredients | 25 % | 50 % | 75 % |
| light mineral oil | 5 % | 5 % | 5 % |
| highly dispersed silicic acid | 5 % | 5 % | - |
| Kaolin | 65 % | 40 % | - |
| Talcum | - | | 20% |

The combination is thoroughly mixed with the adjuvants and the mixture is thoroughly ground in a suitable mill, affording powders that can be used directly for seed treatment.

### Emulsifiable concentrate

| | |
|---|---|
| active ingredients | 10 % |
| octylphenol polyethylene glycol ether (4-5 mol of ethylene oxide) | 3 % |
| calcium dodecylbenzenesulfonate | 3 % |
| castor oil polyglycol ether (35 mol of ethylene oxide) | 4 % |
| Cyclohexanone | 30 % |
| xylene mixture | 50 % |

Emulsions of any required dilution, which can be used in plant protection, can be obtained from this concentrate by dilution with water.

| **Dusts** | **a)** | **b)** | **c)** |
|---|---|---|---|
| Active ingredients | 5 % | 6 % | 4 % |
| Talcum | 95 % | - | - |
| Kaolin | - | 94 % | - |
| mineral filler | - | - | 6 % |

Ready-for-use dusts are obtained by mixing the combination with the carrier and grinding the mixture in a suitable mill. Such powders can also be used for dry dressings for seed.

### Extruder granules

| | |
|---|---|
| Active ingredients | 15 % |
| sodium lignosulfonate | 2 % |
| carboxymethylcellulose | 1 % |
| Kaolin | 82 % |

The combination is mixed and ground with the adjuvants, and the mixture is moistened with water. The mixture is extruded and then dried in a stream of air.

### Coated granules

| | |
|---|---|
| Active ingredients | 8 % |
| polyethylene glycol (mol. wt. 200) | 3 % |
| Kaolin | 89 % |

The finely ground combination is uniformly applied, in a mixer, to the Kaolin moistened with polyethylene glycol. Non-dusty coated granules are obtained in this manner.

### Suspension concentrate

| | |
|---|---|
| active ingredients | 40 % |
| propylene glycol | 10 % |
| nonylphenol polyethylene glycol ether (15 mol of ethylene oxide) | 6 % |
| Sodium lignosulfonate | 10 % |
| carboxymethylcellulose | 1 % |
| silicone oil (in the form of a 75 % emulsion in water) | 1 % |
| Water | 32 % |

The finely ground combination is intimately mixed with the adjuvants, giving a suspension concentrate from which suspensions of any desired dilution can be obtained by dilution with water. Using such dilutions, living plants as well as plant propagation material can be treated and protected against infestation by microorganisms, by spraying, pouring or immersion.

### Flowable concentrate for seed treatment

| | |
|---|---|
| active ingredients | 40 % |
| propylene glycol | 5 % |
| copolymer butanol PO/EO | 2 % |
| Tristyrenephenole with 10-20 moles EO | 2 % |
| 1,2-benzisothiazolin-3-one (in the form of a 20% solution in water) | 0.5 % |
| monoazo-pigment calcium salt | 5 % |
| Silicone oil (in the form of a 75 % emulsion in water) | 0.2 % |
| Water | 45.3 % |

The finely ground combination is intimately mixed with the adjuvants, giving a suspension concentrate from which suspensions of any desired dilution can be obtained by dilution with water. Using such dilutions, living plants as well as plant propagation material can be treated and protected against infestation by microorganisms, by spraying, pouring or immersion.

### Slow Release Capsule Suspension

28 parts of the combination are mixed with 2 parts of an aromatic solvent and 7 parts of toluene diisocyanate/polymethylene-polyphenylisocyanate-mixture (8:1). This mixture is emulsified in a mixture of 1.2 parts of polyvinylalcohol, 0.05 parts of a defoamer and 51.6 parts of water until the desired particle size is achieved. To this emulsion a mixture of 2.8 parts 1,6-diaminohexane in 5.3 parts of water is added. The mixture is agitated until the polymerization reaction is completed. The obtained capsule suspension is stabilized by adding 0.25 parts of a thickener and 3 parts of a dispersing agent. The capsule suspension formulation contains 28% of the active ingredients. The medium capsule diameter is 8-15 microns. The resulting formulation is applied to seeds as an aqueous suspension in an apparatus suitable for that purpose.

The composition of the present may further comprise at least one additional pesticide. For example, the compounds according to the invention can also be used in combination with other herbicides or plant growth regulators. In a preferred embodiment the additional pesticide is a herbicide and/or herbicide safener.

Thus, compounds of formula (I) can be used in combination with one or more other herbicides to provide various herbicidal mixtures. Specific examples of such mixtures include (wherein "I" represents a compound of formula (I)):- I + acetochlor; I + acifluorfen-sodium; I + aclonifen; I + alachlor; I + alloxydim; I + ametryn; I + amicarbazone; I + amidosulfuron; I + aminocyclopyrachlor ; I + aminopyralid; I + amitrole; I + asulam; I + atrazine; I + bensulfuron-methyl; I + bentazone; I + bicyclopyrone; I + bifenox; I + bispyribac-sodium; I + bromacil; I + bromoxynil; I + butafenacil; I + cafenstrole; I + carfentrazone-ethyl; I + chlorimuron-ethyl; I + chlorotoluron; I + cinosulfuron; I + clethodim; I + clodinafop-propargyl; I + clomazone; I + clopyralid; I + cyhalofop-butyl; I + 2,4-D (including the choline salt and 2-ethylhexyl ester thereof); I + daimuron; I + desmedipham; I + dicamba (including the aluminum, aminopropyl, bis-aminopropylmethyl, choline, diglycolamine, dimethylamine, dimethylammonium, potassium and sodium salts thereof); I + diclofop-methyl; I + difenzoquat; I + diflufenican; I + diflufenzopyr; I + dimethachlor; I + dimethenamid-P; I + diquat dibromide; I + diuron; I + esprocarb; I + ethofumesate; I + fenoxaprop-P-ethyl; I + fenquinotrione; I + flazasulfuron; I + florasulam; I + fluazifop-P-butyl; I + flucarbazone-sodium; I + flufenacet; I + flumetralin; I + flumetsulam; I + flumioxazin; I + flupyrsulfuron-methyl-sodium; I + fluroxypyr-meptyl; I + fluthiacet-methyl; I + fomesafen; I + foramsulfuron; I + glufosinate (including the ammonium salt thereof); I + glyphosate (including the diammonium, isopropylammonium and potassium salts thereof); I + halauxifen-methyl; I + halosulfuron-methyl; I + haloxyfop-methyl; I + hexazinone; I + imazamox; I + imazapic; I + imazapyr; I + imazaquin; I + imazethapyr; I + indaziflam; I + iodosulfuron-methyl-sodium; I + iofensulfuron; I + iofensulfuron-sodium; I + ioxynil; I + ipfencarbazone; I + isoxaben; I + isoxaflutole; I + lactofen; I + linuron; I + mecoprop-P; I + mefenacet; I + mesosulfuron; I + mesosulfuron-methyl; I + mesotrione; I + metamitron; I + metobromuron; I + metolachlor; I + metoxuron; I + metribuzin; I + metsulfuron; I + molinate; I + napropamide; I + nicosulfuron; I + norflurazon; I + orthosulfamuron; I + oxadiargyl; I + oxadiazon; I + oxyfluorfen; I + paraquat dichloride; I + pendimethalin; I + penoxsulam; I + phenmedipham; I + picloram; I + picolinafen; I + pinoxaden; I + pretilachlor; I + primisulfuron-methyl; I + prodiamine; I + prometryn; I + propachlor; I + propanil; I + propaquizafop; I + propham; I + propyzamide; I + prosulfocarb; I + prosulfuron; I + pyrasulfotole; I + pyrazolynate, I + pyrazosulfuron-ethyl; I + pyribenzoxim; I + pyridate; I + pyriftalid; I + pyrithiobac-sodium; I + pyroxasulfone; I + pyroxsulam ; I + quinclorac; I + quizalofop-P-ethyl; I + rimsulfuron; I + saflufenacil; I + sethoxydim; I + S-metolachlor; I + sulcotrione; I + sulfentrazone; I + tebuthiuron; I + tefuryltrione; I + tembotrione; I + terbuthylazine; I + terbutryn; I + thiencarbazone; I + thifensulfuron; I + tiafenacil; I + tolpyralate; I + topramezone; I + tralkoxydim; I + triafamone; I + triasulfuron; I + tribenuron-methyl; I + triclopyr; I + trifloxysulfuron-sodium; I + trifludimoxazin and tritosulfuron.

Especially preferred examples of such mixtures include:- I + ametryn; I + atrazine; I + bicyclopyrone; I + butafenacil; I + chlorotoluron; I + clodinafop-propargyl; I + clomazone; I + 2,4-D (including the choline salt and 2-ethylhexyl ester thereof); I + dicamba (including the aluminum, aminopropyl, bis-aminopropylmethyl, choline, diglycolamine, dimethylamine, dimethylammonium, potassium and sodium salts thereof); I + dimethachlor; I + diquat dibromide; I + fluazifop-P-butyl; I + flumetralin; I + fomesafen; I + glufosinate-ammonium; I + glyphosate (including the diammonium, isopropylammonium and potassium salts thereof); I + mesotrione; I + molinate; I + napropamide; I + nicosulfuron; I + paraquat dichloride; I + pinoxaden; I + pretilachlor; I + primisulfuron-methyl; I + prometryn; I + prosulfocarb; I + prosulfuron; I + pyridate; I + pyriftalid; I + pyrazolynate, I + S-metolachlor; I + terbuthylazine; I + terbutryn; I + tralkoxydim; I + triasulfuron and I + trifloxysulfuron-sodium.

Preferred herbicide mixture products for weed control in cereals (especially wheat and/or barley) include:- I + amidosulfuron; I + aminopyralid; I + bromoxynil; I + carfentrazone-ethyl; I + chlorotoluron; I + clodinafop-propargyl; I + clopyralid; I + 2,4-D (including the choline salt and 2-ethylhexyl ester thereof); I + dicamba (including the aluminum, aminopropyl, bis-aminopropylmethyl, choline, diglycolamine, dimethylamine, dimethylammonium, potassium and sodium salts thereof); I + difenzoquat; I + diflufenican; I + fenoxaprop-P-ethyl; I + florasulam; I + flucarbazone-sodium; I + flufenacet; flupyrsulfuron-methyl-sodium; I + fluroxypyr-meptyl; I + halauxifen-methyl; I + iodosulfuron-methyl-sodium; I + iofensulfuron; I + iofensulfuron-sodium; I + mesosulfuron; I + mesosulfuron-methyl; I + metsulfuron; I + pendimethalin; I + pinoxaden; I + prosulfocarb; I + pyrasulfotole; I + pyroxasulfone; I + pyroxsulam; I + topramezone; I + tralkoxydim; I + triasulfuron and I + tribenuron-methyl.

Preferred herbicide mixture products for weed control in corn include:- I + acetochlor; I + alachlor; I + atrazine; I + bicyclopyrone; I + 2,4-D (including the choline salt and 2-ethylhexyl ester thereof); I + dicamba (including the aluminum, aminopropyl, bis-aminopropylmethyl, choline, diglycolamine, dimethylamine, dimethylammonium, potassium and sodium salts thereof); I + diflufenzopyr; I + dimethenamid-P; I + flumioxazin; I + fluthiacet-methyl; I + foramsulfuron; I + glufosinate (including the ammonium salt thereof); I + glyphosate (including the diammonium, isopropylammonium and potassium salts thereof); I + isoxaflutole; I + mesotrione; I + nicosulfuron; I + primisulfuron-methyl; I + prosulfuron; I + pyroxasulfone; I + rimsulfuron; I + S-metolachlor, I + terbutylazine; I + tembotrione; I + thiencarbazone and I + thifensulfuron.

Preferred herbicide mixture products for weed control in rice include:- I + 2,4-D; I + 2,4-D choline salt; I + 2,4-D-2-ethylhexyl ester; I + bensulfuron-methyl; I + bispyribac-sodium; I + cafenstrole; I + cinosulfuron; I + clomazone; I + cyhalofop-butyl; I + daimuron; I + dicamba (including the aluminum, aminopropyl, bis-aminopropylmethyl, choline, diglycolamine, dimethylamine, dimethylammonium, potassium and sodium salts thereof); I + esprocarb; I + fenoxaprop-P-ethyl; I + florasulam; I + halauxifen-methyl; I + halosulfuron-methyl; I + iofensulfuron; I + ipfencarbazone; I + mefenacet; I + mesotrione; I + metsulfuron; I + molinate; I + orthosulfamuron; I + oxadiargyl; I + oxadiazon; I + pendimethalin; I + penoxsulam; I + pretilachlor; I + pyrazolynate, I + pyrazosulfuron-ethyl; I + pyribenzoxim; I + pyriftalid; I + quinclorac; I + tefuryltrione; I + triafamone and I + triasulfuron.

Preferred herbicide mixtures for weed control in soybean include:- I + acifluorfen-sodium; I + ametryn; I + atrazine; I + bentazone; I + bicyclopyrone; I + bromoxynil; I + carfentrazone-ethyl; I + chlorimuron-ethyl; I + clethodim; I + clomazone; I + 2,4-D (including the choline salt and 2-ethylhexyl ester thereof); I + dicamba (including the aluminum, aminopropyl, bis-aminopropylmethyl, choline, diglycolamine, dimethylamine, dimethylammonium, potassium and sodium salts thereof); I + diquat dibromide; I + diuron; I + fenoxaprop-P-ethyl; I + fluazifop-P-butyl; I + flufenacet; I + flumioxazin; I + fomesafen; I + glufosinate (including the ammonium salt thereof); I + glyphosate (including the diammonium, isopropylammonium and potassium salts thereof); I + imazethapyr; I + lactofen; I + mesotrione; I + metolachlor; I + metribuzin; I + nicosulfuron; I + oxyfluorfen; I + paraquat dichloride; I + pendimethalin; I + pyroxasulfone; I + quizalofop-P-ethyl; I + saflufenacil; I + sethoxydim; I + S-metolachlor and I + sulfentrazone.

The mixing partners of the compound of Formula (I) may also be in the form of esters or salts, as mentioned e.g. in The Pesticide Manual, Fourteenth Edition, British Crop Protection Council, 2006.

The compound of formula (I) can also be used in mixtures with other agrochemicals such as fungicides, nematicides or insecticides, examples of which are given in The Pesticide Manual.

The mixing ratio of the compound of formula (I) to the mixing partner is preferably from 1: 100 to 1000:1.

The mixtures can advantageously be used in the above-mentioned formulations (in which case "active ingredient" relates to the respective mixture of compound of formula (I) with the mixing partner).

Compounds of formula (I) of the present invention may also be combined with herbicide safeners. Preferred combinations (wherein "I" represents a compound of formula (I)) include:- I + benoxacor, I + cloquintocet-mexyl; I + cyprosulfamide; I + dichlormid; I + fenchlorazole-ethyl; I + fenclorim; I + fluxofenim; 1+ furilazole I + isoxadifen-ethyl; I + mefenpyr-diethyl; I + N-(2-methoxybenzoyl)-4-[(methylaminocarbonyl)amino] benzenesulfonamide and I + oxabetrinil.

Particularly preferred are mixtures of a compound of formula (I) with cyprosulfamide, isoxadifen-ethyl, cloquintocet-mexyl and/or N-(2-methoxybenzoyl)-4-[(methylaminocarbonyl)amino]benzenesulfonamide.

The safeners of the compound of formula (I) may also be in the form of esters or salts, as mentioned e.g. in The Pesticide Manual, 14th Edition (BCPC), 2006. The reference to cloquintocet-mexyl also applies to a lithium, sodium, potassium, calcium, magnesium, aluminium, iron, ammonium, quaternary ammonium, sulfonium or phosphonium salt thereof as disclosed in WO 02/34048, and the reference to fenchlorazole-ethyl also applies to fenchlorazole, etc.

Preferably the mixing ratio of compound of formula (I) to safener is from 100:1 to 1:10, especially from 20:1 to 1:1.

The mixtures can advantageously be used in the above-mentioned formulations (in which case "active ingredient" relates to the respective mixture of compound of formula (I) with the safener).

The compounds of formula (I) of this invention are useful as herbicides. The present invention therefore further comprises a method for controlling unwanted plants comprising applying to the said plants or a locus comprising them, an effective amount of a compound of the invention or a herbicidal composition containing said compound. 'Controlling' means killing, reducing or retarding growth or preventing or reducing germination. Generally the plants to be controlled are unwanted plants (weeds). 'Locus' means the area in which the plants are growing or will grow.

The rates of application of compounds of formula (I) may vary within wide limits and depend on the nature of the soil, the method of application (pre- or post-emergence; seed dressing; application to the seed furrow; no tillage application etc.), the crop plant, the weed(s) to be controlled, the prevailing climatic conditions, and other factors governed by the method of application, the time of application and the target crop. The compounds of Formula (I) according to the invention are generally applied at a rate of from 10 to 2000 g/ha, especially from 50 to 1000 g/ha.

The application is generally made by spraying the composition, typically by tractor mounted sprayer for large areas, but other methods such as dusting (for powders), drip or drench can also be used.

Useful plants in which the composition according to the invention can be used include crops such as cereals, for example barley and wheat, cotton, oilseed rape, sunflower, maize, rice, soybeans, sugar beet, sugar cane and turf.

Crop plants can also include trees, such as fruit trees, palm trees, coconut trees or other nuts. Also included are vines such as grapes, fruit bushes, fruit plants and vegetables.

Crops are to be understood as also including those crops which have been rendered tolerant to herbicides or classes of herbicides (e.g. ALS-, GS-, EPSPS-, PPO-, ACCase- and HPPD-inhibitors) by conventional methods of breeding or by genetic engineering. An example of a crop that has been rendered tolerant to imidazolinones, e.g. imazamox, by conventional methods of breeding is Clearfield® summer rape (canola). Examples of crops that have been rendered tolerant to herbicides by genetic engineering methods include e.g. glyphosate- and glufosinate-resistant maize varieties commercially available under the trade names RoundupReady® and LibertyLink®. In a particularly preferred aspect, the crop plant has been engineered to over-express homogentisate solanesyltransferase as taught in, for example, WO2010/029311.

Crops are also to be understood as being those which have been rendered resistant to harmful insects by genetic engineering methods, for example Bt maize (resistant to European corn borer), Bt cotton (resistant to cotton boll weevil) and also Bt potatoes (resistant to Colorado beetle). Examples of Bt maize are the Bt 176 maize hybrids of NK® (Syngenta Seeds). The Bt toxin is a protein that is formed naturally by *Bacillus thuringiensis* soil bacteria. Examples of toxins, or transgenic plants able to synthesise such toxins, are described in EP-A-451 878, EP-A-374 753, WO 93/07278, WO 95/34656, WO 03/052073 and EP-A-427 529. Examples of transgenic plants comprising one or more genes that code for an insecticidal resistance and express one or more toxins are KnockOut® (maize), Yield Gard® (maize), NuCOTIN33B® (cotton), Bollgard® (cotton), NewLeaf® (potatoes), NatureGard® and Protexcta®. Plant crops or seed material thereof can be both resistant to herbicides and, at the same time, resistant to insect feeding ("stacked" transgenic events). For example, seed can have the ability to express an insecticidal Cry3 protein while at the same time being tolerant to glyphosate.

Crops are also to be understood to include those which are obtained by conventional methods of breeding or genetic engineering and contain so-called output traits (e.g. improved storage stability, higher nutritional value and improved flavour).

Other useful plants include turf grass for example in golf-courses, lawns, parks and roadsides, or grown commercially for sod, and ornamental plants such as flowers or bushes.

Compounds of formula I and compositions of the invention can typically be used to control a wide variety of monocotyledonous and dicotyledonous weed species. Examples of monocotyledonous species that can typically be controlled include *Alopecurus myosuroides, Avena fatua, Brachiaria plantaginea, Bromus tectorum, Cyperus esculentus, Digitaria sanguinalis, Echinochloa crus-galli, Lolium perenne, Lolium multiflorum, Panicum miliaceum,* Poa *annua, Setaria viridis, Setaria faberi and Sorghum bicolor.* Examples of dicotyledonous species that can be controlled include *Abutilon theophrasti, Amaranthus retroflexus, Bidens pilosa, Chenopodium album, Euphorbia heterophylla, Galium aparine, Ipomoea hederacea, Kochia scoparia, Polygonum convolvulus, Sida spinosa, Sinapis arvensis, Solanum nigrum, Stellaria media, Veronica persica and Xanthium strumarium.* Weeds can also include plants which may be considered crop plants but which are growing outside a crop area ('escapes'), or which grow from seed left over from a previous planting of a different crop ('volunteers'). Such volunteers or escapes may be tolerant to certain other herbicides.

Various aspects and embodiments of the present invention will now be illustrated in more detail by way of example. It will be appreciated that modification of detail may be made without departing from the scope of the invention.

### PREPARATION EXAMPLES

### Example 1 Preparation of 7-(2-benzyloxy-3,6-dichloro-phenyl)-8-hydroxy-5,5-dimethyl-quinoxalin-6-one

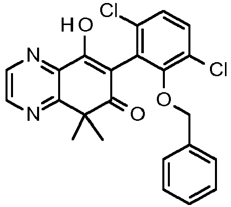

### 1.1 2-Allyl-3,6-dichloro-phenol

A mixture of 2-allyloxy-1,4-dichloro-benzene (1.0 g, 4.9 mmol) and DMF (0.1 mL) was heated at an external temperature of 220 °C for 1 hour. The mixture was allowed to cool to room temperature and was concentrated *in vacuo* to provide 2-allyl-3,6-dichloro-phenol as a brown oil (0.99 g, 99 %). 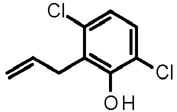 ¹H NMR (400 MHz, CDCl₃): δ_{H}: 7.18-7.08 (m, 1H) 6.95-6.85 (m, 1H) 6.02-5.84 (m, 1H) 5.71 (s, 1H) 5.14-4.99 (m, 2H) 3.59 (dt, 2H).

### 1.2 3,6-Dichloro-2-[(E)-prop-1-enyl]phenol

Potassium *tert*-butoxide (43.6 g, 369 mmol) was added to a solution of 2-allyl-3,6-dichloro-phenol (30.0 g, 148 mmol) in dimethylsulfoxide (150 mL) and the mixture was heated at 56 °C (internal temperature) overnight. The mixture was allowed to cool to room temperature and then poured into an aqueous solution of HCl (150 mL, 2.0 M) at 0 °C. The mixture warmed to room temperature and was further acidified to pH 1 by addition of an aqueous solution of HCl (100 mL, 2.0 M) followed by conc. HCl (10 mL). The mixture was extracted with Et₂O (3 × 100 mL) and the combined organic extracts were dried over MgSO₄, filtered and concentrated *in vacuo.* The crude product was purified by flash column chromatography to provide 3,6-dichloro-2-[(E)-prop-1-enyl]phenol (27.8 g, 93%) as a white solid. 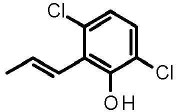 ¹H NMR (400 MHz, CDCl₃): δ_{H}: 7.11 (d, 1H) 6.92 (d, 1H) 6.58-6.44 (m, 2H) 5.93 (s, 1H) 1.98 (d, 3H).

### 1.3 3,6-Dichloro-2-hydroxy-benzaldehyde

A solution of 3,6-dichloro-2-[(*E*)-prop-1-enyl]phenol (22.0 g, 108 mmol) in a mixture of dichloromethane (210 mL) and methanol (100 mL) in a 3-necked flask was cooled to -78 °C. Ozone was bubbled through the solution for 4 hours. Air was bubbled through the solution for 10 minutes. The bubbling of gas through the solution was stopped and dimethyl sulfide (59.7 mL, 813 mmol) was added. The mixture was allowed to warm to room temperature and was stirred for 16 h. The mixture was then concentrated *in vacuo.* The residue was dissolved in CH₂Cl₂ (100 mL) and was washed with brine (100 mL). The organic extracts were dried over MgSO₄, filtered and concentrated *in vacuo.* The crude product was purified by recrystallization from ethanol to provide 3,6-dichloro-2-hydroxy-benzaldehyde (7.03 g, 34%) as a yellow solid. 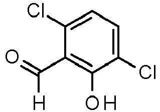 ¹H NMR (400 MHz, CDCl₃): δ_{H}: 12.44 (s, 1H) 10.40 (s, 1H) 7.53 (d, 1H) 6.95 (d, 1H).

### 1.4 2-Benzyloxy-3,6-dichloro-benzaldehyde

Benzyl bromide (1.60 mL, 14.1 mmol) was added to a suspension of 3,6-dichloro-2-hydroxy-benzaldehyde (2.45 g, 12.8 mmol) and potassium carbonate (1.95 g, 14.1 mmol) in a mixture of acetone (64 mL) and *N*,*N*-dimethylformamide (20 mL). The mixture was stirred for 16 hours. The mixture was filtered and the filtrate was concentrated *in vacuo.* The residue was diluted with Et₂O (50 mL) and washed with water (50 mL) then brine (50 mL). The organic extracts were dried over MgSO₄, filtered and concentrated *in vacuo.* The crude product was purified by flash column chromatography to provide 2-benzyloxy-3,6-dichloro-benzaldehyde (2.84 g, 79%) as a pale yellow solid. 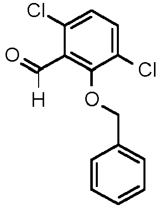 ¹H NMR (400 MHz, CDCl₃): δ_{H}: 10.33 (s, 1H) 7.55 (d, 1H) 7.52 (dd, 2H) 7.47-7.36 (m, 3H) 7.23 (d, 1H) 5.11 (s, 2H).

### 1.5 Diethyl 2-(3-chloropyrazin-2-yl)propanedioate

Diethyl malonate (25.4 mL, 167 mmol) was added to a suspension of 2,3-dichloropyrazine (10.0 g, 67.1 mmol) and potassium carbonate (23.1 g, 167 mmol) in *N,N-*dimethylformamide (80 mL). The reaction mixture was heated at 110 °C for 8 hours then allowed to cool to room temperature. The mixture was filtered and the filtrate was concentrated *in vacuo.* The crude product was purified by flash column chromatography to provide diethyl 2-(3-chloropyrazin-2-yl)propanedioate (12.9 g, 71%). 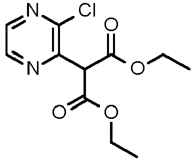 ¹H NMR (400 MHz, CDCl₃): δ_{H}: 8.50 (s, 1H), 8.40 (s, 1H), 5.20 (s, 1H), 4.30 (q, 4H), 1.30 (t, 6H).

### 1.6 2-(3-Chloropyrazin-2-yl)acetic acid

An aqueous solution of NaOH (100 mL, 2.0 M) was added to a solution of diethyl 2-(3-chloropyrazin-2-yl)propanedioate (10.0 g) in ethanol (100 ml). The reaction mixture was heated at 60 °C for 4 hours. The reaction mixture was allowed to cool to room temperature then poured into an aqueous solution of HCl (150 mL, 1.0 M). The mixture was extracted with EtOAc (3 × 100 mL). The combined organic extracts were dried over MgSO₄, filtered and concentrated *in vacuo.* The crude material was purified by trituration with Et₂O to provide 2-(3-chloropyrazin-2-yl)acetic acid (4.35 g, 69 %) as a white solid. 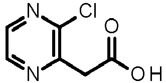 (M-H)⁻ = 171 m/z

### 1.7 Ethyl 2-(3-chloropyrazin-2-yl)acetate

1,1'-carbonyldiimidazole (CDI) was added to a solution of (3-chloropyrazin-2-yl)-acetic acid (2.00 g) in *N*,*N*-dimethylformamide (20 mL) and the mixture stirred for 1 hour. Ethanol (20 mL) was added and the mixture was stirred for 1 hour. The reaction mixture was concentrated *in vacuo* and the crude product was purified by flash column chromatography to provide ethyl 2-(3-chloropyrazin-2-yl)acetate (1.93 g, 83%). 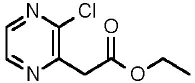 ¹H-NMR (400MHz, CDCl₃): δ_{H}: 8.50 (s, 1H), 8.30 (s, 1H), 4.20 (q, 2H), 4.00 (s, 2H), 1.30 (t, 3H).

### 1.8 Ethyl 2-(3-chloropyrazin-2-yl)-2-methyl-propanoate

A solution of lithium hexamethyldisilazide (7.50 mL, 1.0 M in THF, 7.50 mmol) was added to a solution of ethyl 2-(3-chloropyrazin-2-yl)acetate (1.00 g, 4.98 mmol) in *N,N-*dimethylformamide (10 ml) at 5 °C. Methyl iodide (0.37 mL, 5.98 mmol) was added and the mixture was allowed to warm to room temperature over 30 minutes. The reaction mixture was cooled to 5 °C and a second portion of lithium hexamethyldisilazide (7.50 mL, 1.0 M in THF, 7.50 mmol) was added. Methyl iodide (0.37 mL, 5.98 mmol) was added and the mixture was stirred for 2 hours. The reaction was quenched by addition of an aqueous solution of HCl (10 mL, 1.0 M) then diluted with H₂O (10 mL) and extracted with CH₂Cl₂ (3 × 20 mL). The combined organic extracts were dried over MgSO₄, filtered and concentrated *in vacuo.* The crude product was purified by flash column chromatography to provide ethyl 2-(3-chloropyrazin-2-yl)-2-methylpropanoate (0.70 g, 61%). 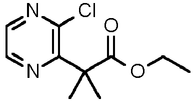 ¹H-NMR (400MHz, CDCl₃): δ_{H}: 8.46 (s, 1H), 8.26 (s, 1H), 4.19-4.14 (m, 2H), 1.64 (s, 6H), 1.19 (t, J=7.04, 3H).

### 1.9 2-benzyloxy-1,4-dichloro-3-ethynyl-benzene

To a stirred suspension of K₂CO₃ (7.40 g, 53.4 mmol) in acetonitrile (250 mL) was added tosyl azide (4.22 g, 3.3 mL, 21.4 mmol) dropwise. 1-dimethoxyphosphorylpropan-2-one [CAS number: 4202-14-6] (3.60 g, 3.0 mL, 21.4 mmol) was then added and the mixture stirred for 3 h at ambient temperature. The reaction was then cooled to 0 °C and a solution of 2-Benzyloxy-3,6-dichloro-benzaldehyde (5.0 g, 17.8 mmol) in MeOH (100mL) was added dropwise. After completion of the addition the reaction mixture was stirred at ambient temperature for 18 h.

The mixture was evaporated to dryness and partitioned between water and ethyl acetate. The organics were kept, washed with brine, dried over Na₂SO₄, filtered and evaporated to yield crude product. Purification by flash column chromatography gave 2-benzyloxy-1,4-dichloro-3-ethynyl-benzene (2.2 g, 45 %). 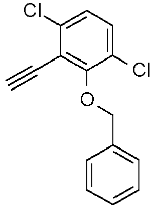 ¹H NMR (400 MHz, CDCl₃): δ_{H}: 7.55 (d, J=7.0, 2H), 7.41-7.35 (m, 3H), 7.31 (d, J=8.7, 1H), 7.14 (d, J=8.7, 1H), 5.14 (s, 2H), 3.64 (s, 1H).

### 1.10 ethyl 2-[3-[2-(2-benzyloxy-3,6-dichloro-phenyl)ethynyl]pyrazin-2-yl]-2-methyl-propanoate

A mixture of ethyl 2-(3-chloropyrazin-2-yl)-2-methyl-propanoate (810 mg, 3.54 mmol), CS₂CO₃ (3.5 g, 10.62 mmol) and acetonitrile (10 mL) was degassed with argon for 15 min. PdCl₂(dppf) (130 mg, 0.177 mmol) and Xantphos (154 mg, 0.266 mmol) were added and the mixture again degassed with argon for 15 min. 2-benzyloxy-1,4-dichloro-3-ethynyl-benzene (1.47 g, 5.31 mmol) was added and the reaction then heated at a temperature between 80 °C and 85 °C for 18 h under argon atmosphere. The reaction was monitored by TLC and consumption of the alkyne observed. The mixture was then filtered to remove solids and evaporated to dryness to obtain a crude product. Purification by flash column chromatography gave ethyl 2-[3-[2-(2-benzyloxy-3,6-dichloro-phenyl)ethynyl]pyrazin-2-yl]-2-methyl-propanoate (351 mg, 22 %). 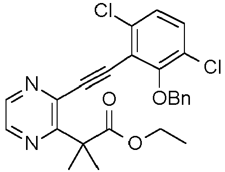 ¹H NMR (400 MHz, CDCl₃): δ_{H}: 8.51-8.48 (m, 2H), 7.55-7.54 (m, 2H), 7.35 (d, J=8.7, 1H), 7.30-7.28 (m, 3H), 7.17 (d, J=8.64, 1H), 5.24 (s, 2H), 4.05-3.99 (m, 2H), 1.69 (s, 6H), 1.02 (t, J=7.12, 3H).

### 1.11 2-[3-[2-(2-benzyloxy-3,6-dichloro-phenyl)ethynyl]pyrazin-2-yl]-2-methyl-propanoic acid

To a stirred solution of ethyl 2-[3-[2-(2-benzyloxy-3,6-dichloro-phenyl)ethynyl]pyrazin-2-yl]-2-methyl-propanoate (450 mg, 95.87 mmol) in ethanol (4 mL) was added 4M NaOH (2 mL) at ambient temperature. Then reaction mixture was heated to reflux for 10 h. The ethanol was evaporated under reduced pressure. The resulting mixture was diluted with water, acidified to pH 1 with 2M HCl and extracted with ethyl acetate (x 2). The combined organics were washed with brine, dried over Na₂SO₄, filtered and evaporated to obtain crude product. Purification by flash column chromatography gave 2-[3-[2-(2-benzyloxy-3,6-dichloro-phenyl)ethynyl]pyrazin-2-yl]-2-methyl-propanoic acid (180 mg, 43 %) as an off-white solid. 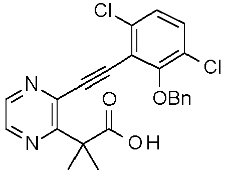 ¹H NMR (400 MHz, DMSO-d6): δ_{H}: 12.59 (s, 1H), 8.68-8.66 (m, 2H), 7.68 (d, J=8.7, 1H), 7.54-7.53 (m, 2H), 7.46 (d, J=8.7, 1H), 7.33-7.32 (m, 3H), 5.19 (s, 2H), 1.64 (s, 6H).

### 1.12 5-[(2-benzyloxy-3,6-dichloro-phenyl)methylene]-8,8-dimethyl-pyrano[3,4-b]pyrazin-7-one

A stirred solution of 2-[3-[2-(2-benzyloxy-3,6-dichloro-phenyl)ethynyl]pyrazin-2-yl]-2-methyl-propanoic acid (40 mg, 0.091 mmol) in dichloromethane (1 mL) was degassed with argon then treated with K₂CO₃ (3 mg, 0.0217 mmol) and AuCl (4 mg, 0.017 mmol). The mixture was stirred at ambient temperature for 18 h. TLC analysis then showed consumption of starting material and the reaction mixture was evaporated to dryness. Purification by flash column chromatography gave 5-[(2-benzyloxy-3,6-dichloro-phenyl)methylene]-8,8-dimethyl-pyrano[3,4-b]pyrazin-7-one (18 mg, 45 %, E/Z geometry not determined). 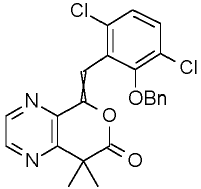 ¹H NMR (400 MHz, DMSO-d6): δ_{H}: 8.76-8.75 (m, 2H), 7.60 (d, J=8.8, 1H), 7.43 (d, J=8.7, 1H), 7.38-7.31 (m, 2H), 7.29-7.20 (m, 3H), 7.07 (s, 1H), 4.93 (s, 2H), 1.46 (s, 6H).

### 1.13 7-(2-benzyloxy-3,6-dichloro-phenyl)-8-hydroxy-5,5-dimethyl-quinoxalin-6-one

To a stirred solution of 5-[(2-benzyloxy-3,6-dichloro-phenyl)methylene]-8,8-dimethyl-pyrano[3,4-b]pyrazin-7-one (150 mg, 0.34 mmol) in acetonitrile (2 mL) was added dried powdered molecular sieves and then triethylamine (103 mg, 142 µL, 1.02 mmol). After stirring at ambient temperature for 10 min, acetone cyanohydrin (17.4 mg, 19 µL, 0.204 mmol) was added and the mixture heated at 50 °C for 24 h. The reaction mass was allowed to cool, diluted with acetonitrile (10 mL) and solids removed by filtration. The resulting solution was concentrated under reduced pressure and the residue partitioned between dichloromethane and 10 %w/v aqueous citric acid. The organic layer was separated and kept, and the aqueous layer re-extracted. The combined organics were washed with brine, dried over Na₂SO₄, filtered and evaporated to obtain a crude residue. Purification by flash column chromatography gave a gum rich in the title compound which was triturated with 10 %v/v ethyl acetate in hexane to obtain 7-(2-benzyloxy-3,6-dichloro-phenyl)-8-hydroxy-5,5-dimethyl-quinoxalin-6-one (40 mg, 20 %) as an off-white solid. 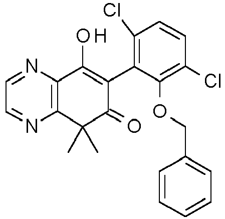 ¹H NMR (400 MHz, CDCl₃): δ_{H}: 8.72 (s, 1H), 8.48 (s, 2H), 7.39 (d, J=8.6, 1H), 7.25-7.19 (m, 3H), 7.08-7.07 (m, 3H), 4.97 (d, J=11.4, 1H), 4.86 (d, J=11.7, 1H), 1.56, s, 3H), 1.44 (s, 3H).

### Example 2 Preparation of 7-(2-benzyloxy-3-chloro-6-fluoro-phenyl)-5,5-dimethyl-6,6-dioxo-thiopyrano[3,4-b]pyrazin-8-ol

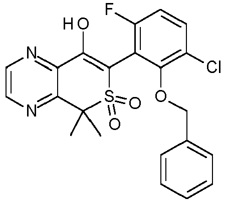

### 2.1 Methyl 3-methylpyrazine-2-carboxylate

To a cooled suspension of 3-methylpyrazine-2-carboxylic acid (5.00 g, 36.2 mmol) in methanol (127 mL) was slowly added concentrated sulfuric acid (13.5 g, 7.34 mL, 127 mmol). The reaction mixture was heated to reflux for 5 h. After this time, LC/MS analysis showed only a single peak, with a mass corresponding to the desired product. The reaction mixture was concentrated *in vacuo.* The resulting residue was dissolved in dichloromethane and washed with excess aqueous 2N NaOH. The organic layer was kept and the aqueous re-extracted with a further 2 portions of dichloromethane. The combined organics were dried over MgSO₄, filtered, and concentrated *in vacuo* to provide the desired ester (3.7 g, 67 %) as a yellow solid. The product was used without further purification in subsequent reactions. 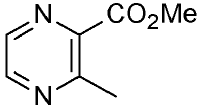 ¹H-NMR (400 MHz, CDCl₃): δ = 8.63 (d, J=2.3 Hz, 1H), 8.53 (d, J=2.0 Hz, 1H), 4.02 (s, 3H), 2.87 (s, 3H).

### 2.2 Methyl 3-(bromomethyl)pyrazine-2-carboxylate

To a stirred suspension of methyl 3-methylpyrazine-2-carboxylate (3.6 g, 24 mmol) in benzotrifluoride (71 mL) was added 1,3-dibromo-5,5-dimethyl-imidazolidine-2,4-dione (3.7 g, 13 mmol) and 2,2'-azodiisobutyronitrile [AIBN] (0.39 g, 0.34 mL, 2.4 mmol). The reaction was heated to reflux and monitored by LC/MS. After 4 h, LC/MS analysis showed consumption of starting material. The reaction was allowed to cool and then concentrated *in vacuo.* The crude product was purified by flash column chromatography to give the desired *bromide* (3.6 g, 16 mmol, 66 %) as a yellow oil. 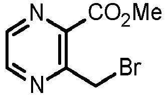 ¹H-NMR (400MHz, CDCl₃): δ = 8.72 (d, J=2.3 Hz, 1H), 8.65 (d, J=2.3 Hz, 1H), 5.04 (s, 2H), 4.12 - 4.02 (m, 3H).

### 2.3 methyl 3-(acetylsulfanylmethyl)pyrazine-2-carboxylate

To a stirred solution of methyl 3-(bromomethyl)pyrazine-2-carboxylate (3.6 g, 16 mmol) in acetone (47 mL) was added potassium thioacetate (2.2 g, 19 mmol). Upon addition, a beige suspension was formed. The reaction mixture was stirred at ambient temperature for 19 h.

After this time the reaction was dark brown. LC/MS analysis showed complete conversion to the desired product. The reaction mixture was filtered through celite, washing with dichloromethane, and concentrated under reduced pressure to give the desired *thioester* (3.5 g, 15 mmol, 99 %) as a brown oil. The product was used in subsequent reactions without further purification. 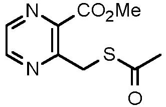 ¹H-NMR (400MHz, CDCl₃): δH = 8.69 (d, J=2.3 Hz, 1H), 8.58 (d, J=2.3 Hz, 1H), 4.70 (s, 2H), 4.07 - 4.02 (m, 3H), 2.39 - 2.31 (m, 3H).

### 2.4 2-allyloxy-1-chloro-4-fluoro-benzene

A solution of 2-chloro-5-fluoro-phenol (100 g, 682 mmol) in acetone (100 mL) was added *via* a dropping funnel to a stirred suspension of potassium carbonate (105 g, 751 mmol) in acetone (1.0 L) at room temperature. The mixture was stirred for 10 min then heated to 50 °C. A solution of allyl bromide (90.8 g, 65.0 mL, 751 mmol) in acetone (100 mL) was added *via* a dropping funnel over 30 min. The mixture was then heated to reflux for 4 h.

GC/MS analysis showed complete consumption of 2-chloro-5-fluoro-phenol. The mixture was cooled to ambient temperature then concentrated *in vacuo.* Solids were removed by filtration, washing with acetone, and the liquors concentrated *in vacuo* to provide a crude orange oil (126.9 g). The crude mixture was purified by vacuum distillation to provide 2-allyloxy-1-chloro-4-fluoro-benzene (108.0 g, 579 mmol, 84.8 %) as a colourless oil. 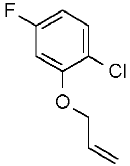 ¹H-NMR (400MHz, CDCl₃): δ_{H}: 7.31 (dd, J = 8.7 and 6.0, 1H), 6.70-6.60 (m, 2H), 6.06 (tdd, J = 17.3, 10.5 and 5.1, 1H), 5.49 (qd, J = 17.3, 1.6, 1H), 5.35 (qd, J = 10.6, 1.4, 1H), 4.60 (td, J = 5.1 and 1.5, 2H).

### 2.5 2-allyl-6-chloro-3-fluoro-phenol

2-allyloxy-1-chloro-4-fluoro-benzene (111 g, 595 mmol) in 1-methylpyrrolidin-2-one (222 mL) was heated at 190 °C (external temperature) for 18 h behind a blast shield. GC/MS analysis showed formation of the desired product. The mixture was allowed to cool to ambient temperature then diluted with EtOAc (300 mL). The mixture was filtered through celite and washed with water (2 x 200 mL) then brine (200 mL). The organics were separated then dried over MgSO₄, filtered and concentrated *in vacuo* to provide a black oil (132 g). The material was dissolved in an aqueous solution of NaOH (300 mL, 2.0 M) then washed with Et₂O (3 x 200 mL). The aqueous layer was kept and acidified to pH 1 by the addition of concentrated hydrochloric acid (80 mL). The mixture was extracted with dichloromethane (3 x 200 mL) and the combined organics dried over MgSO₄, filtered and concentrated *in vacuo* to provide a black oil (109 g). 1H NMR analysis showed a 10:1 mixture of 2-allyl-6-chloro-3-fluoro-phenol : 4-allyl-2-chloro-5-fluoro-phenol. The material was used in subsequent reactions without further purification.
¹H-NMR (400MHz, CDCl₃): δH = 7.16 (1 H, dd, J = 8.9 and 5.7), 6.64 (1 H, t, J = 8.8), 5.96 (1 H, ddt, J = 16.8, 10.4 and 6.2), 5.70 (1H, d, J = 1.3), 5.16-4.99 (2 H, m), 3.46 (2 H, dd, J = 6.2 and 1.5).

### 2.6 3-allyl-2-benzyloxy-1-chloro-4-fluoro-benzene

Benzyl bromide (2.6 g, 1.8 mL, 15 mmol) was added to a suspension of 2-allyl-6-chloro-3-fluoro-phenol (2.5 g, 13 mmol) and potassium carbonate (2.1 g, 15 mmol) in acetone (27 mL) and the mixture was heated at reflux for 16 h.

After this time, TLC analysis showed complete consumption of starting material. The reaction mixture was filtered and the filtrate was concentrated *in vacuo.* The crude product was purified by flash column chromatography to afford 3-allyl-2-benzyloxy-1-chloro-4-fluoro-benzene (2.5 g, 9.0 mmol, 67 %) as a colourless oil. 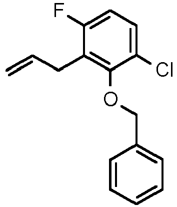 ¹H NMR (400 MHz, CDCl₃): δ = 7.53 - 7.46 (m, 2H), 7.45 - 7.34 (m, 3H), 7.28 - 7.23 (m, 1H), 6.84 (t, J=8.6 Hz, 1H), 5.93 (ddt, 1H), 5.05 - 4.96 (m, 4H), 3.45 - 3.34 (m, 2H).

### 2.7 2-(2-benzyloxy-3-chloro-6-fluoro-phenyl)acetic acid

Ruthenium (III) chloride (0.13 g, 0.64 mmol) was added to a solution of 3-allyl-2-benzyloxy-1-chloro-4-fluoro-benzene (8.9 g, 32 mmol) in a mixture of water (96 mL), acetonitrile (64 mL) and ethyl acetate (64 mL). Sodium periodate (34 g, 160 mmol) was added portionwise (9 portions) over a period of 30 min keeping the internal temperature below 25 °C. LC/MS analysis showed formation of the desired product. The reaction mixture was cooled to 5 °C and a solution of sodium metabisulfite (61 g, 320 mmol) in water (100 mL) was added dropwise over 1 h maintaining the internal temperature below 10 °C. A starch-iodide test for oxidants was negative. The mixture was phase separated and the aqueous layer was extracted with EtOAc (2 x 200 mL). The combined organic extracts were dried over MgSO₄, passed through a hydrophobic frit and concentrated *in vacuo* to provide a brown solid (9.171 g). The crude product was purified by flash column chromatography to provide a white solid (4.94 g). The material was recrystallised from dichloromethane - isohexane to provide the desired *carboxylic acid* (4.199 g, 14.25 mmol, 44 %) as a white solid. 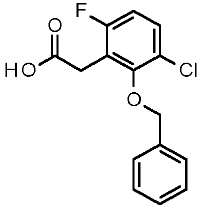 ¹H NMR (400 MHz, CDCl₃): δH = 7.49-7.31 (6 H, m), 6.88 (1 H, t, J = 8.7), 5.05 (2 H, s), 3.68 (2 H, d, J = 1.6).

### 2.8 2-benzyloxy-3-(bromomethyl)-1-chloro-4-fluoro-benzene

To a stirred cooled solution of 2-(2-benzyloxy-3-chloro-6-fluoro-phenyl)acetic acid (1.3 g, 4.4 mmol) in dichloromethane (70 ml) was added oxalyl chloride (3.8 ml, 44 mmol) and one drop of *N*,*N*-dimethylformamide. The reaction was stirred at ambient temperature for 2 h. After completion of the reaction, the mixture was evaporated *in vacuo.* The flask containing the residue was completely covered with aluminium foil to exclude light. Bromotrichloromethane (50 ml) followed by 2-mercapto-pyridine-1-oxide sodium salt (658 mg, 4.4 mmol) were added to the reaction mass in the dark and it was heated at 100 °C for 1 h. The reaction mixture was then allowed to cool, opened to the light and stirred at ambient temperature for 17 h. The mass was diluted with dichloromethane and water. The organic layer was separated, washed with brine, dried over Na₂SO₄, then concentrated under reduced pressure to afford the desired *bromide* (0.69 g, 48 %) as a deep red solid. 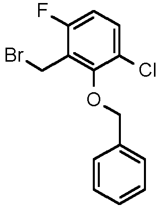 ¹H-NMR (400MHz, CDCl₃): δ = 7.59 - 7.53 (m, 2H), 7.46 - 7.34 (m, 4H), 6.88 (t, J=8.7 Hz, 1H), 5.20 (s, 2H), 4.52 (d, J=1.6 Hz, 2H).

### 2.9 methyl 3-[(2-benzyloxy-3-chloro-6-fluoro-phenyl)methylsulfanylmethyl] pyrazine-2-carboxylate

To a stirred solution of methyl 3-(acetylsulfanylmethyl)pyrazine-2-carboxylate (1.1 g, 4.9 mmol) in methanol (44 mL) was added potassium carbonate (1.3 g, 9.7 mmol). The mixture was stirred at ambient temperature for 10 min. 2-benzyloxy-3-(bromomethyl)-1-chloro-4-fluorobenzene (1.8 g, 5.3 mmol) was added to the reaction mixture and stirring was continued for 3 days.

After this time, the reaction mixture was concentrated under reduced pressure to remove methanol. The residue was dissolved in ethyl acetate and washed with water followed by brine. The organic layer was dried over MgSO₄, filtered, and concentrated *in vacuo.* Purification by flash column chromatography provided the desired *sulfide* (0.76 g, 1.8 mmol, 36 %) as an orange oil. 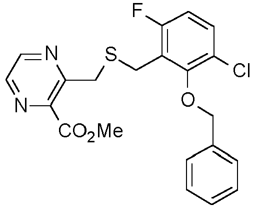 ¹H-NMR (400MHz, CDCl₃): δH = 8.54-8.49 (m, 2H), 7.45 - 7.34 (m, 5H), 7.24 - 7.27 (m, 1H), 6.82 (t, J=8.7 Hz, 1H), 5.06 (s, 2H), 4.34 (s, 2H), 4.01 - 3.95 (m, 3H), 3.83 (d, J=1.5 Hz, 2H).

### 2.10 methyl 3-[(2-benzyloxy-3-chloro-6-fluoro-phenyl)methylsulfonylmethyl]pyrazine-2-carboxylate

To a stirred solution of methyl 3-[(2-benzyloxy-3-chloro-6-fluorophenyl)methylsulfanylmethyl]pyrazine-2-carboxylate (0.76 g, 1.8 mmol) in dichloromethane (19 mL) was added 3-chloroperoxybenzoic acid [mCPBA] (0.91 g, 3.7 mmol). The reaction mixture was stirred at ambient temperature for 17 h.

The reaction was quenched by addition of saturated aqueous sodium hydrogen carbonate solution and saturated sodium thiosulfate solution and the mixture stirred for 30 min. After this time the phases were separated, and the aqueous layer extracted with 2 further portions of dichloromethane. The combined organics were dried over MgSO₄, filtered, and concentrated *in vacuo* to give the desired sulfone (0.80 g, 1.7 mmol, 98%) as a yellow oil. The product was used without further purification in subsequent reactions. 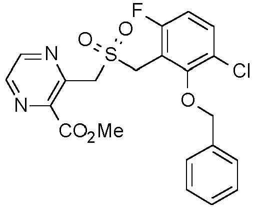 ¹H-NMR (400MHz, CDCl₃): δ = 8.67 - 8.59 (m, 2H), 7.47 - 7.34 (m, 6H), 6.94 (t, J=8.7 Hz, 1H), 5.20 (s, 4H), 4.56 - 4.48 (m, 2H), 3.99 (s, 3H).

### 2.11 7-(2-benzyloxy-3-chloro-6-fluoro-phenyl)-5,5-dimethyl-6,6-dioxo-thiopyrano[3,4-b]pyrazin-8-ol

To a solution of methyl 3-[(2-benzyloxy-3-chloro-6-fluorophenyl)methylsulfonylmethyl]pyrazine-2-carboxylate (0.80 g, 1.7 mmol) in *N,N-*dimethylformamide (6.9 mL) cooled to 0°C under a nitrogen atmosphere was added potassium *tert*-butoxide solution (1.7 mL, 1.7 mmol, 1.0M in tetrahydrofuran) dropwise. The reaction mixture gradually turned yellow and then orange/brown. The reaction was stirred at this temperature for 20 min before addition of iodomethane (0.25 g, 0.11 mL, 1.7 mmol). The mixture was then stirred at 0°C for a further 1 h. After this time, LC/MS analysis showed that mono-methylation had occurred. Additional potassium *tert*-butoxide solution (1.7 mL, 1.7 mmol, 1.0M in tetrahydrofuran) was added, and the reaction stirred for 20 min. Iodomethane (0.25 g, 0.11 mL, 1.7 mmol) was added and the reaction stirred at 0°C for 1 h. Cooling was withdrawn and the mixture stirred for a further 17 h at ambient temperature.

Additional potassium *tert*-butoxide solution (2.1 mL, 2.1 mmol, 1.0M in tetrahydrofuran) was added and the reaction stirred at ambient temperature for a further 3 h. After this time, LC/MS analysis suggested formation of the desired dimethylated, cyclized product. The reaction mixture was partitioned between ethyl acetate and 2M hydrochloric acid. The organic layer was dried over Na₂SO₄ and concentrated *in vacuo* to afford an orange residue. Purification by flash column chromatography gave 7-(2-benzyloxy-3-chloro-6-fluoro-phenyl)-5,5-dimethyl-6,6-dioxothiopyrano[3,4-b]pyrazin-8-ol (0.27 g, 0.59 mmol, 34 %) as a yellow solid. 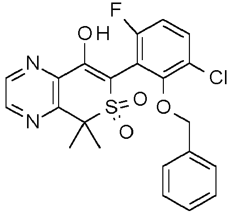 ¹H-NMR (400MHz, CDCl₃): δ = 8.72 (d, J=2.4 Hz, 1H), 8.57 (d, J=24.5 Hz, 1H), 7.50 (dd, J=5.9, 8.9 Hz, 1H), 7.44 (dd, J=2.4, 7.2 Hz, 2H), 7.26 - 7.21 (m, 3H), 6.99 (t, J=7.7 Hz, 1H), 5.20 (s, 2H), 1.85 - 1.83 (m, 3H), 1.80 (s, 3H).

Table 1 below provides 15 specific examples of compounds of formula (I) of the invention.

**TABLE 1**

| **Compound No.** | **Structural Formula** | **¹H NMR details** |
|---|---|---|
| 1.01 | 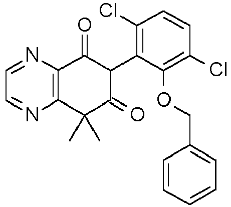 | (400 MHz, CDCl₃) δH ppm: 8.72 (s, 1H), 8.48 (s, 2H), 7.39 (d, J=8.6, 1H), 7.25-7.19 (m, 3H), 7.08-7.07 (m, 3H), 4.97 (d, J=11.5, 1H), 4.86 (d, J=11.5, 1H), 1.56 (s, 3H), 1.44 (s, 3H). |
| 1.02 | 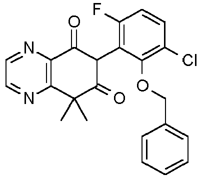 | (400 MHz, CDCl₃) δH ppm: 8.72 (d, 1H), 8.56 (bs, 1H), 8.48 (d, 1H), 7.42 (dd, 1H), 7.27-7.20 (m, 2H), 7.13-7.07 (m, 3H), 6.93 (t, 1H), 5.01 (d, 1H), 4.89 (d, 1H), 1.56 (s, 3H), 1.42 (s, 3H). |
| 1.03 | 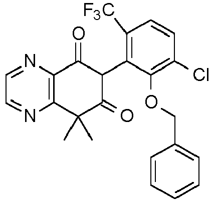 | |
| 1.04 | 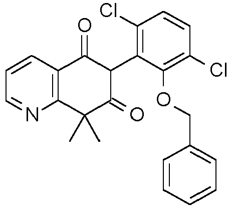 | |
| 1.05 | 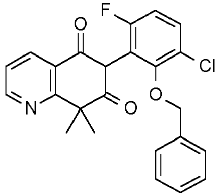 | |
| 1.06 | 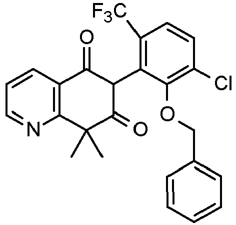 | |
| 1.07 | 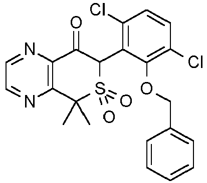 | (400MHz, CDCl₃) δH ppm: 8.73 (d, J=2.4 Hz, 1H), 8.58 (d, J=2.4 Hz, 1H), 7.50 - 7.41 (m, 3H), 7.30 (d, J=8.7 Hz, 1H), 7.26 - 7.22 (m, 3H), 5.28 - 5.21 (m, 1H), 5.19 - 5.11 (m, 1H), 1.86 (s, 3H), 1.82 (s,3H). |
| 1.08 | 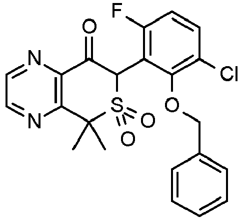 | (400 MHz, CDCl₃) δH ppm: 8.72 (d, J=2.4 Hz, 1H), 8.57 (d, J=24.5 Hz, 1H), 7.50 (dd, J=5.9, 8.9 Hz, 1H), 7.44 (dd, J=2.4, 7.2 Hz, 2H), 7.26 - 7.21 (m, 3H), 6.99 (t, J=7.7 Hz, 1H), 5.20 (s, 2H), 1.85 - 1.83 (m, 3H), 1.80 (s,3H). |
| 1.09 | 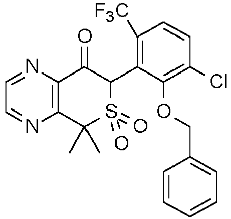 | |
| 1.10 | 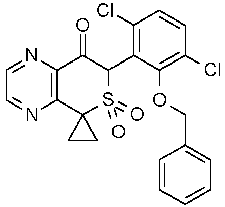 | (400MHz, CDCl₃) δH ppm: 8.63 (d, J=2.2 Hz, 1H), 8.55 (d, J=2.3 Hz, 1H), 7.48 (d, J=8.7 Hz, 1H), 7.40 (dd, J=2.8, 6.4 Hz, 2H), 7.31 (d, J=8.7 Hz, 1H), 7.23 - 7.15 (m, 3H), 5.27 (d, J=10.6 Hz, 1H), 5.04 (d, J=10.6 Hz, 1H), 2.14 - 2.06 (m, 1H), 2.05 - 1.99 (m, 1H), 1.91 - 1.78 (m,2H). |
| 1.11 | 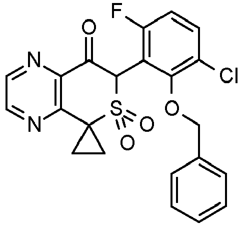 | |
| 1.12 | 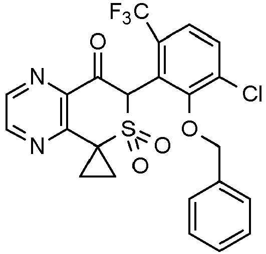 | |
| 1.13 | 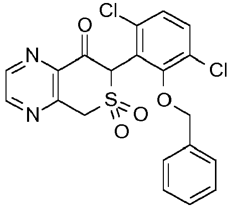 | (400MHz, CDCl₃) δH ppm: 8.74 - 8.58 (m, 2H), 7.53 - 7.43 (m, 1H), 7.41 - 7.35 (m, 2H), 7.31 (d, J=8.7 Hz, 1H), 7.20 - 7.12 (m, 3H), 5.25 (d, J=11.0 Hz, 1H), 5.08 (d, J=11.0 Hz, 1H), 4.71 (d, J=16.1 Hz, 1H), 4.41 (d, J=16.1 Hz, 1H). |
| 1.14 | 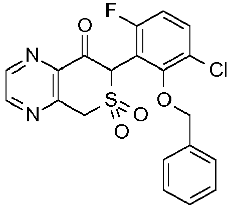 | |
| 1.15 | 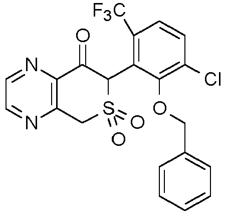 | |

### BIOLOGICAL EXAMPLES

### B1 Post-emergence efficacy

Seeds of a variety of test species are sown in standard soil in pots:- *Solanum nigrum* (SOLNI), *Amaranthus retoflexus* (AMARE), *Setaria faberi* (SETFA), *Echinochloa crus-galli* (ECHCG), *Ipomoea hederacea* (IPOHE), *Lolium perenne* (LOLPE). After 8 days cultivation (post-emergence) under controlled conditions in a glasshouse (at 24/16°C, day/night; 14 hours light; 65% humidity), the plants are sprayed with an aqueous spray solution derived from the formulation of the technical active ingredient in acetone/water (50:50) solution containing 0.5% Tween 20 (polyoxyethylene sorbitan monolaurate, CAS RN 9005-64-5). Compounds are applied at 1000 g/ha and 250 g/ha. The test plants are then grown in a glasshouse under controlled conditions in a glasshouse (at 24/16°C, day/night; 14 hours light; 65 % humidity) and watered twice daily. After 13 days, the test is evaluated for the percentage damage caused to the plant. The biological activities are shown below in Table B1, on a five point scale (5 = 80-100%; 4 = 60-79%; 3=40-59%; 2=20-39%; *1*=0-19%).

**Table B1 Control of weed species by compounds of formula (I) after post-emergence application at a rate of 1000 g/Ha**

| Compound | Application Rate (g/Ha) | SOLNI | AMARE | SETFA | ECHCG | IPOHE | LOLPE |
|---|---|---|---|---|---|---|---|
| 1.01 | 1000 | 5 | 5 | 5 | 5 | 5 | 5 |
| | 250 | 5 | 5 | 5 | 5 | 5 | 5 |
| 1.02 | 1000 | 5 | 5 | 5 | 5 | 5 | 5 |
| | 250 | 5 | 5 | 5 | 5 | 5 | 5 |
| 1.08 | 1000 | 5 | 5 | 5 | 5 | 5 | 5 |
| | 250 | 5 | 5 | 5 | 4 | 5 | 5 |
| 1.13 | 1000 | 5 | 5 | 5 | 5 | 5 | 5 |
| | 250 | 5 | 5 | 5 | 5 | 5 | 5 |

## Claims

1. A compound of formula (I) 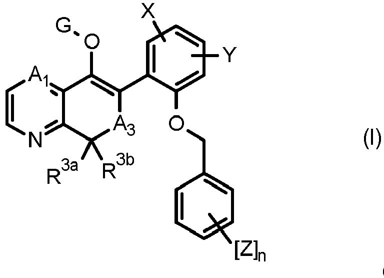 or a salt or N-oxide thereof;
wherein A₁ is CR¹ or N;
R¹ is hydrogen, C₁-C₄alkyl, C₁-C₄haloalkyl, C₁-C₄alkoxy, C₁-C₄alkylthio, halogen, cyano, or hydroxyl;
A₃ is C(O) or S(O)₂;
G is hydrogen, or C(O)R⁶;
X and Y are each independently hydrogen, C₁-C₃alkyl, C₁-C₃alkoxy, C₁-C₃haloalkyl, C₁-C₃haloalkoxy, or halogen;
n is an integer of 0, 1, 2, 3, 4, or 5;
each Z is independently C₁-C₃alkyl, C₁-C₃alkoxy, C₁-C₃haloalkyl, C₁-C₃haloalkoxy, or halogen;
R^{3a} and R^{3b} are independently hydrogen, halogen, cyano, C₁-C₃alkyl, C₁-C₈alkoxy-C₁-C₄alkyl-, C₁-C₈haloalkyl, C₂-C₈alkenyl, C₂-C₈haloalkenyl, C₂-C₈alkynyl, C₂-C₈haloalkynyl, C₃-C₁₀cycloalkyl, C₃-C₁₀cycloalkyl-C₁-C₄alkyl-, heterocyclyl, heterocyclyl-C₁-C₄alkyl-, or C₁-C₈alkoxycarbonyl-; or R^{3a} and R^{3b} together with the carbon atom they are attached to join to form a 3- to 10-membered carbocyclic ring or a 4- to 10-membered heterocyclic ring;
R⁶ is selected from the group consisting of C₁-C₆alkyl, C₂-C₆alkenyl, C₂-C₆alkynyl, C₁-C₆alkyl-S-, -NR⁷R⁸ and phenyl optionally substituted by one or more R⁹;
R⁷ and R⁸ are independently selected from the group consisting of C₁-C₆ alkyl and C₁-C₆ alkoxy, or R⁷ and R⁸ together can form a morpholinyl ring; and,
R⁹ is selected from the group consisting of halogen, cyano, nitro, C₁-C₃alkyl, C₁-C₃haloalky, C₁-C₃alkoxy and C₁-C₃haloalkoxy; and
with the proviso that when A₁ is CR¹, A₃ is C(O).

2. The compound of claim 1, wherein G is hydrogen, or C(O)R⁶ wherein R⁶ is isopropyl, *tert*-butyl, methyl, ethyl, propargyl or methoxy.

3. The compound of claim 1 or claim 2, wherein R^{3a} and R^{3b} are each independently hydrogen, halogen, C₁-C₈alkyl, C₁-C₈haloalkyl or C₂-C₈alkynyl.

4. The compound according to any one of the preceding claims wherein X is C₁-C₃alkyl, C₁-C₃alkoxy, C₁-C₃haloalkyl, C₁-C₃haloalkoxy, or halogen, and is *ortho* with respect to the bi-cyclic moiety.

5. The compound according to any one of the preceding claims wherein Y is C₁-C₃alkyl, C₁-C₃alkoxy, C₁-C₃haloalkyl, C₁-C₃haloalkoxy, or halogen, and is *ortho* with respect to the benzyloxy moiety.

6. The compound according to any one of the preceding claims wherein A₃ is C(O).

7. The compound according to any one of claims 1 to 5 wherein A₃ is S(O)₂.

8. The compound according to any one of the preceding claims wherein R¹ is selected from the group consisting of hydrogen, halogen, C₁-C₃alkyl and C₁-C₃alkoxy.

9. The compound according to any one of the preceding claims wherein n is 0, 1 or 2.

10. The compound according to any one of the preceding claims wherein each Z is independently selected from halogen, methyl, methoxy, trifluoromethyl, and trifluoromethoxy.

11. A herbicidal composition comprising a herbicidal compound according to any one of claims 1 to 10 and an agriculturally acceptable formulation adjuvant.

12. A herbicidal composition according to claim 11, further comprising at least one additional pesticide.

13. A method of controlling unwanted plant growth, comprising applying a compound of formula (I) as defined in any one of claims 1 to 10, or a herbicidal composition according to either one of claims 11 or 12, to the unwanted plants or to the locus thereof.

14. Use of a compound of formula (I) as defined in any one of claims 1 to 10 as a herbicide.

## Patentansprüche

1. Verbindung der Formel (I) 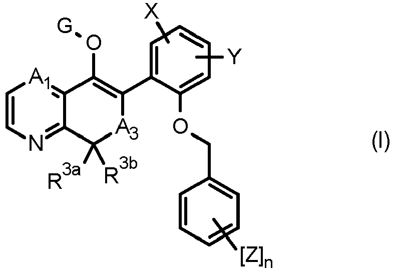 oder ein Salz oder N-Oxid davon,
wobei A₁ für CR¹ oder N steht,
R¹ für Wasserstoff, C₁-C₄-Alkyl, C₁-C₄-Halogenalkyl, C₁-C₄-Alkoxy, C₁-C₄-Alkylthio, Halogen, Cyano oder Hydroxyl steht,
A₃ für C(O) oder S(O)₂ steht,
G für Wasserstoff oder C(O)R⁶ steht,
X und Y jeweils unabhängig voneinander für Wasserstoff, C₁-C₃-Alkyl, C₁-C₃-Alkoxy, C₁-C₃-Halogenalkyl, C₁-C₃-Halogenalkoxy oder Halogen stehen,
n für eine ganze Zahl 0, 1, 2, 3, 4 oder 5 steht,
Z jeweils unabhängig für C₁-C₃-Alkyl, C₁-C₃-Alkoxy, C₁-C₃-Halogenalkyl, C₁-C₃-Halogenalkoxy oder Halogen steht,
R^{3a} und R^{3b} unabhängig voneinander für Wasserstoff, Halogen, Cyano, C₁-C₈-Alkyl, C₁-C₈-Alkoxy-C₁-C₄-alkyl-, C₁-C₈-Halogenalkyl, C₂-C₈-Alkenyl, C₂-C₈-Halogenalkenyl, C₂-C₈-Alkinyl, C₂-C₈-Halogenalkinyl, C₃-C₁₀-Cycloalkyl, C₃-C₁₀-Cycloalkyl-C₁-C₄-alkyl-, Heterocyclyl, Heterocyclyl-C₁-C₄-alkyl- oder C₁-C₈-Alkoxycarbonyl- stehen oder R^{3a} und R^{3b} zusammen mit dem Kohlenstoffatom, an das sie gebunden sind, einen 3- bis 10-gliedrigen carbocyclischen Ring oder einen 4- bis 10-gliedrigen heterocyclischen Ring bilden,
R⁶ aus der aus C₁-C₆-Alkyl, C₂-C₆-Alkenyl, C₂-C₆-Alkinyl, C₁-C₆-Alkyl-S-, -NR⁷R⁸ und Phenyl, gegebenenfalls substituiert durch ein oder mehrere R⁹, bestehenden Gruppe ausgewählt ist,
R⁷ und R⁸ unabhängig voneinander aus der aus C₁-C₆-Alkyl und C₁-C₆-Alkoxy bestehenden Gruppe ausgewählt sind oder R⁷ und R⁸ zusammen einen Morpholinylring bilden können und
R⁹ aus der aus Halogen, Cyano, Nitro, C₁-C₃-Alkyl, C₁-C₃-Halogenalkyl, C₁-C₃-Alkoxy und C₁-C₃-Halogenalkoxy bestehenden Gruppe ausgewählt ist und
mit der Maßgabe, dass, wenn A₁ für CR¹ steht, A₃ für C(O) steht.

2. Verbindung nach Anspruch 1, wobei G für Wasserstoff oder C(O)R⁶ steht, wobei R⁶ für Isopropyl, tert.-Butyl, Methyl, Ethyl, Propargyl oder Methoxy steht.

3. Verbindung nach Anspruch 1 oder Anspruch 2, wobei R^{3a} und R^{3b} jeweils unabhängig voneinander für Wasserstoff, Halogen, C₁-C₈-Alkyl, C₁-C₈-Halogenalkyl oder C₂-C₈-Alkinyl stehen.

4. Verbindung nach einem der vorhergehenden Ansprüche, wobei X für C₁-C₃-Alkyl, C₁-C₈-Alkoxy, C₁-C₃Halogenalkyl, C₁-C₃-Halogenalkoxy oder Halogen steht und orthoständig in Bezug auf die bicyclische Gruppierung ist.

5. Verbindung nach einem der vorhergehenden Ansprüche, wobei Y für C₁-C₈-Alkyl, C₁-C₃-Alkoxy, C₁-C₃Halogenalkyl, C₁-C₃-Halogenalkoxy oder Halogen steht und orthoständig in Bezug auf die Benzyloxygruppierung ist.

6. Verbindung nach einem der vorhergehenden Ansprüche, wobei A₃ für C(O) steht.

7. Verbindung nach einem der Ansprüche 1 bis 5, wobei A₃ für S(O)₂ steht.

8. Verbindung nach einem der vorhergehenden Ansprüche, wobei R¹ aus der aus Wasserstoff, Halogen, C₁-C₃-Alkyl und C₁-C₃-Alkoxy bestehenden Gruppe ausgewählt ist.

9. Verbindung nach einem der vorhergehenden Ansprüche, wobei n für 0, 1 oder 2 steht.

10. Verbindung nach einem der vorhergehenden Ansprüche, wobei Z jeweils unabhängig aus Halogen, Methyl, Methoxy, Trifluormethyl und Trifluormethoxy ausgewählt ist.

11. Herbizide Zusammensetzung, umfassend eine herbizide Verbindung nach einem der Ansprüche 1 bis 10 und ein landwirtschaftlich unbedenkliches Formulierungshilfsmittel.

12. Herbizide Zusammensetzung nach Anspruch 11, ferner umfassend mindestens ein zusätzliches Pestizid.

13. Verfahren zur Bekämpfung von unerwünschtem Pflanzenwuchs, bei dem man eine wie in einem der Ansprüche 1 bis 10 definierte Verbindung der Formel (I) oder eine herbizide Zusammensetzung nach Anspruch 11 oder 12 auf die unerwünschten Pflanzen oder deren Standort ausbringt.

14. Verwendung einer wie in einem der Ansprüche 1 bis 10 definierten Verbindung der Formel (I) als Herbizid.

## Revendications

1. Composé de formule (I) 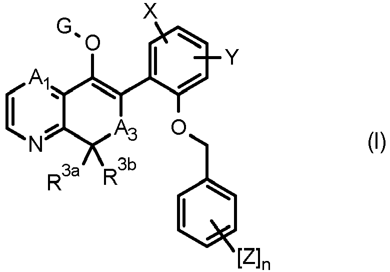 ou un sel ou N-oxyde de celui-ci ;
dans lequel A₁ est CR¹ ou N ;
R¹ est hydrogène, C₁-C₄alkyle, C₁-C₄halogénoalkyle, C₁-C₄alcoxy, C₁-C₄alkylthio, halogène, cyano, ou hydroxyle ;
A₃ est C(O) ou S(O)₂ ;
G est hydrogène, ou C(O)R⁶ ;
X et Y sont chacun indépendamment hydrogène, C₁-C₃alkyle, C₁-C₃alcoxy, C₁-C₃halogénoalkyle, C₁-C₃halogénoalcoxy, ou halogène ;
n est un nombre entier valant 0, 1, 2, 3, 4, ou 5 ;
chaque Z est indépendamment C₁-C₃alkyle, C₁-C₃alcoxy, C₁-C₃halogénoalkyle, C₁-C₃halogénoalcoxy, ou halogène ;
R^{3a} et R^{3b} sont indépendamment hydrogène, halogène, cyano, C₁-C₈alkyle, C₁-C₈alcoxy-C₁-C₄alkyl-, C₁-C₈halogénoalkyle, C₂-C₈alcényle, C₂-C₈halogénoalcényle, C₂-C₈alcynyle, C₂-C₈halogénoalcynyle, C₃-C₁₀cycloalkyle, C₃-C₁₀cycloalkyl-C₁-C₄alkyl-, hétérocyclyle, hétérocyclyl-C₁-C₄alkyl-, ou C₁-C₈alcoxycarbonyl- ; ou R^{3a} et R^{3b}, conjointement avec l'atome de carbone auquel ils sont fixés, forment un cycle carbocyclique de 3 à 10 chaînons ou un cycle hétérocyclique de 4 à 10 chaînons ;
R⁶ est choisi dans le groupe constitué par C₁-C₆alkyle, C₂-C₆alcényle, C₂-C₆alcynyle, C₁-C₆alkyl-S-, - NR⁷R⁸ et phényle éventuellement substitué par un ou plusieurs R⁹ ;
R⁷ et R⁸ sont choisis indépendamment dans le groupe constitué par C₁-C₆ alkyle et C₁-C₆ alcoxy, ou R⁷ et R⁸ peuvent former ensemble un cycle morpholinyle ; et,
R⁹ est choisi dans le groupe constitué par halogène, cyano, nitro, C₁-C₃alkyle, C₁-C₃halogénoalkyle, C₁-C₃alcoxy et C₁-C₃halogénoalcoxy ; et
à condition que lorsque A₁ est CR¹, A₃ soit C (O).

2. Composé selon la revendication 1, dans lequel G est hydrogène, ou C(O)R⁶ où R⁶ est isopropyle, tertio-butyle, méthyle, éthyle, propargyle ou méthoxy.

3. Composé selon la revendication 1 ou la revendication 2, dans lequel R^{3a} et R^{3b} sont chacun indépendamment hydrogène, halogène, C₁-C₈alkyle, C₁-C₈halogénoalkyle ou C₂-C₈alcynyle.

4. Composé selon l'une quelconque des revendications précédentes, dans lequel X est C₁-C₃alkyle, C₁-C₃alcoxy, C₁-C₃halogénoalkyle, C₁-C₃halogénoalcoxy, ou halogène, et est *ortho* par rapport au motif bicyclique.

5. Composé selon l'une quelconque des revendications précédentes, dans lequel Y est C₁-C₃alkyle, C₁-C₃alcoxy, C₁-C₃halogénoalkyle, C₁-C₃halogénoalcoxy, ou halogène, et est *ortho* par rapport au motif benzyloxy.

6. Composé selon l'une quelconque des revendications précédentes, dans lequel A₃ est C(O).

7. Composé selon l'une quelconque des revendications 1 à 5, dans lequel A₃ est S(O)₂.

8. Composé selon l'une quelconque des revendications précédentes, dans lequel R¹ est choisi dans le groupe constitué par hydrogène, halogène, C₁-C₃alkyle et C₁-C₃alcoxy.

9. Composé selon l'une quelconque des revendications précédentes, dans lequel n vaut 0, 1 ou 2.

10. Composé selon l'une quelconque des revendications précédentes, dans lequel chaque Z est choisi indépendamment parmi halogène, méthyle, méthoxy, trifluorométhyle, et trifluorométhoxy.

11. Composition herbicide comprenant un composé herbicide selon l'une quelconque des revendications 1 à 10 et un adjuvant de formulation acceptable sur le plan agricole.

12. Composition herbicide selon la revendication 11, comprenant en outre au moins un pesticide supplémentaire.

13. Méthode de contrôle d'une croissance végétale non désirée, comprenant l'application d'un composé de formule (I), tel que défini selon l'une quelconque des revendications 1 à 10, ou d'une composition herbicide selon la revendication 11 ou bien 12, aux plantes non désirées ou au lieu où elles se développent.

14. Utilisation d'un composé de Formule (I) tel que défini selon l'une quelconque des revendications 1 à 10, comme herbicide.
